# EUROPEAN PATENT APPLICATION

(11) **EP 4 706 580 A2**
(43) Date of publication of application: **11.03.2026**
(21) Application number: 25226638.2
(22) Date of filing: 29.05.2024
(51) Int. Cl.: A61B 90/00

(54) **SURGICAL INSTRUMENT, AND GUIDE ASSEMBLY AND DISASSEMBLING TOOL THEREFOR**

(30) Priority: 02.06.2023 CN 202310651971; 02.08.2023 CN 202310971324; 27.02.2024 CN 202410215043
(62) Divisional of application: 24814506.2
(71) Applicant: Hangzhou Mindray Medical Technology Co., Ltd., Hangzhou, Zhejiang 311508 (CN)
(72) Inventor: CHENG, Jiaqing, Hangzhou, 311508 (CN); WU, Hekun, Hangzhou, 311508 (CN); CHEN, Rongqin, Hangzhou, 311508 (CN); YANG, Weiwang, Hangzhou, 311508 (CN)
(74) Representative: KIPA AB

(57) **Abstract**

A surgical instrument, and a guide assembly and a disassembling tool therefor are disclosed. The disassembling tool includes a frame body and a movement mechanism which is arranged at the frame body. A forward movement of the frame body along the first path is capable of triggering the movement mechanism to move relative to the guide assembly along the second path. A reverse movement of the frame body along the first path is capable of triggering the movement mechanism to drive the guide assembly and the effector assembly of the surgical instrument to switch from a joined state to a separate state. The guide assembly can be disassembled in two simple steps. The operation is simple and efficient, which greatly facilitates disassembling and replacement of the guide assembly.

## Description

This application requests for respective priorities of following three Chinese patent applications: application date: June 2, 2023, application number: 202310651971.1; application date: August 2, 2023, application number: 202310971324.9; application date: February 27, 2024, application number: 202410215043.5. The entire contents of above applications are hereby incorporated into this disclosure by reference.

### TECHNICAL FIELD

The disclosure relates to a technical field of medical instrument, and more particularly to a surgical instrument, and a guide assembly and a disassembling tool therefor.

### BACKGROUND

At present, a minimally invasive surgery occupies an important position in surgeries, and a surgical stapling instrument is a commonly used surgical instrument in a minimally invasive surgery. An existing surgical stapling instrument includes an operation handle and an end effector. The operation handle has a fixed handle for an operator to hold, and the operation handle is connected with the end effector through a slender body. A working principle of the surgical stapling instrument is to clamp a designated position, which is adjacent to a lesion site, by jaws of an end effector, excise tissue through a cutting knife of the end effector, and stitch through a staple cartridge assembly of the end effector while excising.

However, in actual surgical scenarios, some surgical sites are special and an end of an effector assembly is difficult to extend around tissue to be cut, such as difficult-to-reach blood vessels. At this time, an ordinary surgical stapling instrument cannot satisfy a surgical requirement, and the operator often has to use a device which is dedicated to vascular anastomosis, so as to satisfy a clinical use, which greatly increases medical costs and expenses. Therefore, there is an urgent need for a surgical instrument which is capable of anastomosing a normal tissue and facilitating entry into a smaller space.

### SUMMARY

A main object of this disclosure is to provide a guide assembly, which is capable of being detachably connected with an end of an effector assembly of a surgical instrument, so as to enable the end of the effector assembly of the surgical instrument to enter into a smaller space; as well as a surgical instrument with the guide assembly, and a disassembling tool for disassembling the guide assembly from the end of the effector assembly.

In one aspect, in an embodiment, a guide assembly for a surgical instrument is provided, including:
a main-body member, which includes a joining portion and a guide portion, wherein the joining portion is joined with an end of an effector assembly of the surgical instrument, and the guide portion is arranged at the joining portion;
a movable position-limiting member, which is in movable connection with the joining portion or the guide portion, and includes a slidable snap-fit portion, wherein the movable position-limiting member is configured to switch the joining portion and the end of the effector assembly between a separate state and a joined state;
wherein, when the joining portion is joined with the end of the effector assembly, the slidable snap-fit portion is in slidable contact with a slidable guide portion of the effector assembly, so as to slide along a guide direction of the slidable guide portion; while the slidable snap-fit portion slides, the movable position-limiting member generates a non-flexible deformation when abutting against the slidable guide portion, and releases the non-flexible deformation when the slidable snap-fit portion slides to a joining position, so as to snap-fit with a snap-fit structure of the effector assembly, wherein the guide direction of the slidable guide portion intersects with a joining direction of the effector assembly.

In an embodiment, the slidable snap-fit portion protrudes from the movable position-limiting member, and is provided with a first guide surface; wherein the slidable snap-fit portion is in slidable contact with the slidable guide portion of the effector assembly through the first guide surface, and the first guide surface is parallel to the guide direction.

In an embodiment, the joining portion and/or the guide portion are/is provided with a first position-limiting portion, wherein the first position-limiting portion(s) is(are) arranged along a movement path of the movable position-limiting member; wherein, when the slidable snap-fit portion slides inclinedly along the guide direction, the first position-limiting portion(s) squeezes(squeeze) the movable position-limiting member, so as to enable the movable position-limiting member to generate the non-flexible deformation.

In an embodiment, the first position-limiting portion(s) is(are) provided with an inclined second guide surface, wherein the movable position-limiting member slides along the second guide surface, so as to generate the non-flexible deformation.

In an embodiment, the first position-limiting portions are respectively arranged on both sides of the main-body member, wherein the first position-limiting portions on both sides of the main-body member are configured to jointly squeeze the movable position-limiting member, so as to generate the non-flexible deformation, which deformation expands outward to store energy.

In an embodiment, the first position-limiting portion(s) is(are) configured to squeeze the movable position-limiting member, so as to generate the non-flexible deformation, which deformation expands outward to store energy; wherein, when the slidable snap-fit portion slides to the joining position, the movable position-limiting member contracts to release the non-flexible deformation, which deformation expands outward to store energy.

In an embodiment, the movable position-limiting member includes a first cantilever, a second cantilever and a connection arm; wherein one end of the first cantilever is in movable connection with one side of the main-body member, the other end of the first cantilever is connected with the connection arm; one end of the second cantilever is in movable connection with the other side of the main-body member, the other end of the second cantilever is connected with the connection arm; wherein the slidable snap-fit portion is arranged at the connection arm.

In an embodiment, the joining portion and/or the guide portion are/is provided with a second position-limiting portion, wherein the second position-limiting portion(s) is(are) configured to limit a movement distance of the movable position-limiting member.

In an embodiment, the joining portion and/or the guide portion are/is provided with a movement groove, wherein one end of the movable position-limiting member is in movable connection with the movement groove(s), and an edge of the movement groove extends to form the second position-limiting portion, or respective edges of the movement grooves extend to form the second position-limiting portions.

In an embodiment, a length of the guide portion along the joining direction is 6-9 cm; and/or
the guide portion is a curved structure, and a ratio of a length of the guide portion along the joining direction to a height which is formed by the curve structure is 0.8-2; and/or
one end of the guide portion, which end is away from the joining portion, is a tip structure; and an angle, which is formed by extended surfaces of both sides of the tip structure intersecting, is 10-25°.

In an embodiment, a length of the guide portion along the joining direction is 7-8 cm; and/or
the guide portion is a curved structure, and a ratio of a length of the guide portion along the joining direction to a height which is formed by the curve structure is 1-1.5; and/or
one end of the guide portion, which end is away from the joining portion, is a tip structure; and an angle, which is formed by extended surfaces of both sides of the tip structure intersecting, 15-20°.

In an embodiment, the joining portion is provided with a guide channel, wherein, when the joining portion and the end of the effector assembly are in the joined state, the guide channel is connected with a knife slot of the effector assembly.

In an embodiment, the guide channel includes a guide groove.

In an embodiment, the joining portion is provided with two spaced-apart joining arms at one end, which end is away from the guide portion, wherein the joining arms are inserted into a plug-in slot of the effector assembly, and a space between the two joining arms forms the guide channel; and/or
the joining portion is a C-shaped structure or an H-shaped structure; and/or
the joining portion is provided with a snap-fit opening; wherein, when the joining portion and the end of the effector assembly are in the joined state, a portion of the slidable snap-fit portion penetrates through the snap-fit structure and is located inside the snap-fit opening.

In one aspect, in an embodiment, a guide assembly for a surgical instrument is provided, including:
a main-body member, which includes a joining portion and a guide portion, wherein the joining portion is joined with an end of an effector assembly, and the guide portion is arranged at the joining portion;
a movable position-limiting member, which is in movable connection with the joining portion or the guide portion, and is provided with a slidable snap-fit portion, wherein the movable position-limiting member is capable of moving relative to the main-body member, so as to switch the joining portion and the end of the effector assembly between a separate state and a joined state;
the joining portion is provided with a guide channel, wherein, when the joining portion and the end of the effector assembly are in the joined state, the guide channel is connected with a knife slot of the effector assembly.

In an embodiment, the guide channel includes a guide groove.

In an embodiment, the joining portion is provided with two spaced-apart joining arms at one end, which end is away from the guide portion, wherein the joining arms are connected with a plug-in slot of the effector assembly, and a space between the two joining arms forms the guide channel; and/or
the joining portion is a C-shaped structure or an H-shaped structure.

In an embodiment, a disassembling tool for a guide assembly for a surgical instrument is provided, including:
a frame body, which implements a forward movement and a reverse movement along a first path relative to the guide assembly for the surgical instrument and an effector assembly of the surgical instrument, while the disassembling tool disassembles the guide assembly;
a movement mechanism, which is in movable connection with the frame body; wherein the forward movement of the frame body along the first path is capable of triggering the movement mechanism to move relative to the guide assembly along a second path, and the reverse movement of the frame body along the first path is capable of triggering the movement mechanism to drive the guide assembly and the effector assembly of the surgical instrument to switch from a joined state to a separate state.

In an embodiment, the first path is a straight path, which is parallel to a joining direction of the guide assembly, and the second path is a circular path, wherein the straight path intersects with the circular path.

In an embodiment, when the frame body implements the forward movement along the first path, the guide assembly passes over the movement mechanism and drives the movement mechanism to implement a forward movement along the second path; when the guide assembly is in position, the guide assembly is located on one side of the movement mechanism, and the movement mechanism abuts against the effector assembly.

In an embodiment, when the frame body implements the reverse movement along the first path, the movement mechanism abuts against a movable position-limiting member of the guide assembly and drives the movable position-limiting member to move, so as to switch the movable position-limiting member and the effector assembly from the joined state to the separate state.

In an embodiment, the movement mechanism includes a separating member and a powering member, the forward movement of the frame body along the first path is capable of driving the separating member to implement a forward movement along the second path, and the powering member is configured to apply a force on the separating member, so as to drive the separating member to implement a reverse movement along the second path to abut against a knife slot of the effector assembly; the reverse movement of the frame body along the first path is capable of triggering the separating member to abut against a movable position-limiting member of the guide assembly and drive the movable position-limiting member to move, so as to switch the movable position-limiting member and the effector assembly from the joined state to the separate state.

In an embodiment, the separating member includes a switching lever, which is provided with a connection end and an effector end, which ends are opposite with each other; wherein the connection end is in rotatable connection with the frame body, and the effector end is configured to switch the movable position-limiting member and the effector assembly from the joined state to the separate state; and/or
the powering member includes a torsion spring, and the powering member applies a torsion force to the separating member, so as to drive the separating member to implement the reverse movement along the second path.

In an embodiment, the effector end is provided with a hook-shaped structure, which is configured to drive the movable position-limiting member to move.

In an embodiment, the frame body is provided with a third position-limiting portion, which is configured to limit an extreme position for the reverse movement of the separating member along the second path, so as to provide a gap between the separating member and a bottom surface of the knife slot of the effector assembly, when the separating member is inserted into the knife slot.

In an embodiment, the separating member is provided with a fourth position-limiting portion, which abuts against the effector assembly, so as to provide a gap between the separating member and a bottom surface of the knife slot of the effector assembly, when the separating member is inserted into the knife slot.

In an embodiment, the frame body is provided with a fifth position-limiting portion, which is configured to limit an extreme position for a reverse movement of the guide assembly along the first path, so as to position the guide assembly on an inner side of the movement mechanism.

In an embodiment, the frame body includes an accommodation cavity, wherein the movement mechanism is arranged inside the accommodation cavity; the frame body is provided with a first opening, through which the guide assembly enters into and exits from the accommodation cavity, so as to enable the guide assembly to move inside the accommodation cavity along the first path; wherein the frame body is further provided with a second opening, through which the guide assembly is taken out from the frame body after the guide assembly is separated from the effector assembly.

In an embodiment, a surgical instrument is provided, including:
a handle assembly;
a barrel assembly, wherein a proximal end of the barrel assembly is connected with the handle assembly;
an effector assembly, wherein the effector assembly is connected with an end of the barrel assembly, the effector assembly includes a staple anvil and a staple cartridge base, wherein the staple anvil is in movable connection with the staple cartridge base, the staple anvil and the staple cartridge base are capable of moving relative to each other to close and open; and
the guide assembly described above.

In an embodiment, the staple anvil is provided with a plug-in slot, a snap-fit structure and a slidable guide portion, wherein the plug-in slot and the snap-fit structure are arranged along a length direction of the staple anvil, the plug-in slot is located at an end of the staple anvil, and the slidable guide portion extends from the plug-in slot to the snap-fit structure.

In an embodiment, the slidable guide portion is a third guide surface, which is parallel to a guide direction.

In an embodiment, the staple anvil is further provided with a T-shaped knife slot, wherein the plug-in slot, the snap-fit structure and the knife slot are connected in sequence; and/or the snap-fit structure is a circular hole, which is perpendicular to a joining direction.

In an embodiment, a distance between a surface of one end of the movable position-limiting member and an assembling surface of the joining portion is smaller than a maximum distance between a surface of the staple anvil and a bottom surface of the plug-in slot, wherein the one end of the movable position-limiting member is provided with the slidable snap-fit portion; the surface of the one end of the movable position-limiting member back-faces the joining portion; and the surface of the staple anvil back-faces the staple cartridge base.

In an embodiment, the above-mentioned disassembling tool is also included.

In an embodiment, a packaging assembly is further included, wherein the packaging assembly includes a first packaging member and a second packaging member, wherein the first packaging member is configured to package the handle assembly, the barrel assembly and the effector assembly, and the second packaging member is configured to package the guide assembly and the disassembling tool.

In an embodiment, the second packaging member includes an outer housing and an inner liner, wherein the outer housing is provided with an inner cavity, and the inner liner is detachably arranged inside the inner cavity of the outer housing, wherein the inner liner is configured to accommodate at least one of the guide assembly and the disassembling tool.

In an embodiment, the inner liner is provided with a first assembling groove and a second assembling groove, wherein the first assembling groove is configured to assemble the guide assembly, and the second assembling groove is configured to assemble the disassembling tool.

In an embodiment, a structure for taking and placing is located at a joint between the inner liner and the outer housing, wherein the structure for taking and placing is configured to take the inner liner out from the outer housing and place the inner liner into the outer housing.

In an embodiment, a surgical instrument is provided, including:
a handle assembly;
a barrel assembly, wherein a proximal end of the barrel assembly is connected with the handle assembly;
an effector assembly, wherein the effector assembly is connected with an end of the barrel assembly;
a guide assembly, which is connected with the effector assembly;
a disassembling tool, which is configured to drive the guide assembly and the effector assembly of the surgical instrument to switch from a joined state to a separate state; and
a packaging assembly, which includes a second packaging member, wherein the second packaging member is configured to package the guide assembly and the disassembling tool; wherein the second packaging member includes an outer housing and an inner liner, wherein the outer housing is provided with an inner cavity, the inner liner is arranged inside the inner cavity of the outer housing and capable of being taken out from the inner cavity; wherein the inner liner is configured to package at least one of the guide assembly and the disassembling tool; wherein the inner liner has a sterile environment.

According to a surgical instrument, a guide assembly and a disassembling tool thereof in an above-mentioned embodiment, the guide assembly includes a main-body member and a movable position-limiting member. The main-body member includes a joining portion and a guide portion. The joining portion is joined with an end of an effector assembly of the surgical instrument, and the guide portion is arranged at the joining portion. The guide portion is capable of guiding the effector assembly of the surgical instrument to be inserted into a narrower space for performing a surgical operation. When it is necessary to operate on tissue in a relatively narrow space, the operator can join the joining portion of the guide assembly with the end of the effector assembly of the surgical instrument, so as to guide the effector assembly into the tissue in the narrow space through the guide portion of the guide assembly. When the guide assembly is not needed, the operator can operate the movable position-limiting member to move, so as to switch the guide assembly and the surgical instrument from a joined state to a separate state, and then disassemble the guide assembly. The operator is capable of satisfying a surgical requirement with only one surgical instrument with a guide assembly, without having to operate another instrument, which is conducive to reducing medical costs and expenses.

The movable position-limiting member of the guide assembly is in movable connection with the joining portion or the guide portion, and the movable position-limiting member can move relative to the main-body member, so as to switch the joining portion and the end of the effector assembly between a separate state and a joined state. When the movable position-limiting member is subjected to a force along a joining direction, the movable position-limiting member generates a non-flexible deformation when abutting against the slidable guide portion, and releases the non-flexible deformation when the slidable snap-fit portion slides to a joining position, so as to snap-fit with a snap-fit structure of the effector assembly, wherein the guide direction of the slidable guide portion intersects with a joining direction of the effector assembly. In other words, the joining portion and the end of the effector assembly can be snap-fitted by means of elastic sliding, that is, a one-step snap-fit can be achieved, and an efficiency for the snap-fit between the joining portion and the end of the effector assembly is high and the operation is simple.

The joining portion of the guide assembly may be provided with a guide channel. Wherein, when the joining portion and the end of the effector assembly are in the joined state, the guide channel is connected with a knife slot of the effector assembly. In such a way, a cutting knife in the knife slot can extend into the guide channel of the joining portion, thereby avoiding interference with a movement of the cutting knife when the joining portion is joined with the end of the effector assembly.

The disassembling tool of a surgical instrument includes a frame body and a movement mechanism which is arranged at the frame body. A forward movement of the frame body along the first path is capable of triggering the movement mechanism to move relative to the guide assembly along the second path. A reverse movement of the frame body along the first path is capable of triggering the movement mechanism to drive the guide assembly and the effector assembly of the surgical instrument to switch from a joined state to a separate state. In other words, the forward movement of the frame body along the first path and the reverse movement along the first path can separate the guide assembly from the effector assembly, that is, the guide assembly can be disassembled in two simple steps. The operation is simple and efficient, which greatly facilitates disassembling and replacement of the guide assembly.

A packaging assembly for a surgical instruments includes a second packaging member, which includes an outer housing and an inner liner which is located inside the outer housing, the inner liner is configured to package at least one of a guide assembly and a disassembling tool; when an operator uses the packaging assembly, the inner liner, and the guide assembly and/or the disassembling tool packaged by the inner liner can be taken out together, the inner liner plays a positioning and guiding role, making it more convenient for the operator to interact with the guide assembly and/or the disassembling tool, and can avoid a problem that the guide assembly and/or the disassembling tool are placed separately, causing the operator to have difficulty finding them. Moreover, the inner liner has a sterile environment, which can avoid the guide assembly and/or the disassembling tool being directly placed in a sterile environment, such as an operating table, during use, thereby avoiding cross-contamination between the guide assembly and/or the disassembling tool and the sterile environment.

On the other hand, in an embodiment, a guide assembly for a surgical instrument is provided, including:
a main-body member, which includes a joining portion and a guide portion, wherein the joining portion is joined with an end of an effector assembly of the surgical instrument, and the guide portion is arranged at the joining portion;
a movable position-limiting member, which is in movable connection with the joining portion or the guide portion, wherein the movable position-limiting member is capable of moving relative to the main-body member, so as to switch the joining portion and the end of the effector assembly between a separate state and a joined state;
wherein, when the movable position-limiting member is subjected to a force along a first direction, the joining portion and the end of the effector assembly are switched from the joined state to the separate state; wherein the first direction is different from a direction of a reaction force, which force is applied by a working object to the end of the effector assembly during working.

In an embodiment, an angle between the first direction and the direction of the reaction force is b, wherein a value range of the angle b is: 90°≤b≤180°.

In an embodiment, the first direction is opposite to the direction of the reaction force.

In an embodiment, the movable position-limiting member is provided with snap-fit portion(s), the main-body member and/or the effector assembly are/is provided with a snap-fit structure, which is snap-fitted with the snap-fit portion; wherein the snap-fit portion(s) is(are) snap-fitted with the snap-fit structure(s) in the joined state.

In an embodiment, the snap-fit portion is configured as a snap-fit column; the snap-fit structure includes a first butt joint hole, which perforates through the joining portion, and a second butt joint hole, which is arranged at the end of the effector assembly; wherein in the joined state, the snap-fit column penetrates through the first butt joint hole and the second butt joint hole, so as to join and then lock the joining portion with the end of the effector assembly.

In an embodiment, the snap-fit portion is configured as a snap-fit rod, wherein in the joined state, the snap-fit rod is snap-fitted with a snap-fit groove at the end of the effector assembly.

In an embodiment, the joining portion is a plug-in structure, which is plugged into and fitted with a plug-in slot at the end of the effector assembly.

In an embodiment, the movable position-limiting member is capable of rotating relative to the main-body member.

In an embodiment, the main-body member is provided with a rotation-shaft hole, and the movable position-limiting member is provided with a rotation-shaft portion, wherein the rotation-shaft portion fits with the rotation-shaft hole, so as to enable the movable position-limiting member to rotate relative to the main-body member through the rotation-shaft portion.

In an embodiment, a side portion of the main-body member is provided with a first position-limiting portion and a second position-limiting portion which portions protrude thereat; wherein in the joined state, the first position-limiting portion and the second position-limiting portion fit with the movable position-limiting portion, so as to prevent the movable position-limiting portion from moving relative to the main-body member.

In an embodiment, two opposite side portions of the main-body member are respectively provided with a rotation-shaft hole, and the movable position-limiting member is provided with two rotation-shaft portions, each portion fits with the rotation-shaft hole in a one-to-one correspondence, so as to enable the movable position-limiting member to rotate relative to the main-body member through the two rotation-shaft portions;
two opposite sides of the main-body member are respectively provided with a first position-limiting portion and a second position-limiting portion which portions protrude thereat; wherein in the joined state, the first position-limiting portion and the second position-limiting portion fit with the movable position-limiting portion, so as to prevent the movable position-limiting portion from moving relative to the main-body member.

In an embodiment, an elastic fitting portion is provided on one side of the movable position-limiting member, which side faces the main-body member, wherein the elastic fitting portion is capable of being elastically deformed under an external force, so as to enable the movable position-limiting member and the main-body member to switch between a fixed state and a movable state.

In an embodiment, the main-body member and the end of the effector assembly are joined to form a smooth contact surface for tissue.

In an embodiment, a guide assembly for a surgical instrument is provided, including:
a main-body member, which includes a joining portion and a guide portion, wherein the joining portion is joined with an end of an effector assembly of the surgical instrument, and the guide portion is arranged at the joining portion;
a movable position-limiting member, which is in movable connection with the main-body member, wherein the movable position-limiting member is capable of moving relative to the main-body member, so as to switch the joining portion and the end of the effector assembly between a separate state and a joined state; wherein the movable position-limiting member is provided with an assembling portion and a disassembling portion, wherein the disassembling portion and the assembling portion are located at different positions of the movable position-limiting member; wherein in the joined state, the movable position-limiting member is configured to join and lock the joining portion with the end of the effector assembly; the disassembling portion is configured to fit with a disassembling tool, so as to switch the joining portion and the end of the effector assembly from the joined state to the separate state.

In an embodiment, the main-body member and/or the effector assembly are/is provided with a snap-fit structure which fits with the assembling portion; wherein the assembling portion is snap-fitted with the snap-fit structure(s) in the joined state.

In an embodiment, the assembling portion is configured as a snap-fit column; the snap-fit structure includes a first butt joint hole, which perforates through the joining portion, and a second butt joint hole, which is arranged at the end of the effector assembly; wherein in the joined state, the snap-fit column penetrates through the first butt joint hole and extends to the second butt joint hole, so as to join and then lock the joining portion with the end of the effector assembly.

In an embodiment, a surgical instrument is provided, including:
a handle assembly;
a barrel assembly, wherein a proximal end of the barrel assembly is connected with the handle assembly;
an effector assembly, wherein the effector assembly is connected with a distal end of the barrel assembly, the effector assembly includes a staple anvil and a staple cartridge base, wherein the staple anvil is in movable connection with the staple cartridge base, the staple anvil and the staple cartridge base are capable of moving relative to each other to close and open; and
a guide assembly, which includes a main-body member and a movable position-limiting member; wherein the main-body member includes a joining portion and a guide portion, the joining portion is joined with an end of the effector assembly of the surgical instrument, and the guide portion is arranged at the joining portion;
wherein, the movable position-limiting member is in movable connection with the main-body member, and is capable of moving relative to the main-body member, so as to switch the joining portion and the staple anvil between a separate state and a joined state; wherein the movable position-limiting member is provided with an assembling portion and a disassembling portion, wherein the disassembling portion and the assembling portion are located at different positions of the movable position-limiting member; wherein in the joined state, the movable position-limiting member is configured to join and lock the joining portion with the end of the effector assembly; the disassembling portion is configured to fit with a disassembling tool, so as to switch the joining portion and the end of the effector assembly from the joined state to the separate state.

In an embodiment, a side portion of the staple anvil is provided with a recessed portion, which is recessed inward from an edge of the side portion of the staple anvil; wherein in the joined state, the disassembling portion of the movable position-limiting member extends to the recessed portion; wherein the recessed portion is configured to enable a movement mechanism of a disassembling tool to extend into and contact with the disassembling portion, and configured to drive the movable position-limiting member to move relative to the main-body member, so as to switch the joining portion and the staple anvil from the joined state to the separate state.

In an embodiment, two opposite side portions of the staple anvil are both provided with the recessed portion.

In an embodiment, the recessed portion is configured in a stepped shape or an arc shape.

In an embodiment, the main-body member and/or the effector assembly are/is provided with a snap-fit structure, which is snap-fitted with the assembling portion; wherein the assembling portion is snap-fitted with the snap-fit structure(s) in the joined state.

In an embodiment, the assembling portion is configured as a snap-fit column; the snap-fit structure includes a first butt joint hole, which perforates through the joining portion, and a second butt joint hole, which is arranged at the end of the effector assembly; wherein in the joined state, the snap-fit column penetrates through the first butt joint hole and extends to the second butt joint hole, so as to join and then lock the joining portion with the end of the effector assembly.

In an embodiment, the second butt joint hole is a blind hole, wherein one end of the blind hole, which end is away from the movable position-limiting member, is closed.

In an embodiment, a guide assembly for a surgical instrument is provided, including:
a main-body member, which includes a joining portion and a guide portion, wherein the joining portion is joined with an end of an effector assembly of the surgical instrument, and the guide portion is arranged at the joining portion;
a movable position-limiting member, which is in movable connection with the main-body member, wherein the movable position-limiting member is capable of moving relative to the main-body member, so as to switch the joining portion and the end of the effector assembly between a separate state and a joined state; wherein in the joined state, the joining portion is rigidly connected with the end of the effector assembly, and the movable position-limiting member is located on a surface, on which surface a staple anvil of the effector assembly joins with tissue.

In an embodiment, the movable position-limiting member is provided with snap-fit portion(s), the main-body member and/or the effector assembly are/is provided with a snap-fit structure, which is snap-fitted with the snap-fit portion; wherein the snap-fit portion(s) is(are) snap-fitted with the snap-fit structure(s) in the joined state.

In an embodiment, the snap-fit portion is configured as a snap-fit column; the snap-fit structure includes a first butt joint hole, which perforates through the joining portion, and a second butt joint hole, which is arranged at the end of the effector assembly; wherein in the joined state, the snap-fit column penetrates through the first butt joint hole and the second butt joint hole, so as to join and then lock the joining portion with the end of the effector assembly.

In an embodiment, a disassembling tool for a guide assembly for a surgical instrument is provided, including:
a frame body, which moves along a first path relative to the guide assembly and an effector assembly of the surgical instrument, while the disassembling tool disassembles the guide assembly;
a movement mechanism, which is in movable connection with the frame body; wherein the movement of the frame body along the first path is capable of triggering the movement mechanism to move relative to the guide assembly along a second path, so as to drive the guide assembly and the effector assembly of the surgical instrument to switch from a joined state to a separate state.

In an embodiment, the first path is different from the second path.

In an embodiment, when the frame body moves relative to the effector assembly along the first path, the frame body drives the movement mechanism to contact with the effector assembly and/or the guide assembly, so as to trigger the movement mechanism; when the movement mechanism is triggered, the movement mechanism moves along the second path and applies a continuous force to a movable position-limiting member of the guide assembly, so as to switch the movable position-limiting member and the effector assembly from the joined state to the separate state.

In an embodiment, the movement mechanism is in rotatable connection with the frame body, and the movement mechanism includes a contact portion and an effector portion; wherein, when the frame body moves relative to the effector assembly along the first path, the frame body drives the contact portion to contact with the guide assembly, so as to enable the effector portion to rotate relative to the guide assembly along the second path; wherein the effector portion is configured to apply a force on a movable position-limiting member of the guide assembly, so as to switch the movable position-limiting member and the effector assembly from the joined state to the separate state.

In an embodiment, a rotation-shaft member is further included; wherein the frame body and the movement mechanism are respectively provided with a rotation-shaft hole; wherein the rotation-shaft member penetrates through respective rotation-shaft holes at the frame body and the movement mechanism, so as to achieve the rotatable connection between the movement mechanism and the frame body.

In an embodiment, the effector portion includes a pushing shaft; wherein, when the movement mechanism moves along the second path, the pushing shaft extends into a first butt joint hole of the guide assembly and a second butt joint hole of the effector assembly, so as to separate the movable position-limiting member of the guide assembly from the first butt joint hole and the second butt joint hole.

In an embodiment, the effector portion includes two pushing shafts; wherein, when the movement mechanism moves along the second path, the two pushing shafts extend into two opposite recessed portions at the effector assembly, so as to contact with the movable position-limiting member, drive the movable position-limiting member to move relative to the main-body member, and switch the guide assembly and the effector assembly from the joined state to the separate state.

In an embodiment, the effector portion includes a hook member; wherein, when the movement mechanism moves along the first path, the hook member is configured to hook the movable position-limiting member of the guide assembly, so as to drive the movable position-limiting member of the guide assembly to separate from a first butt joint hole and a second butt joint hole.

In an embodiment, the effector portion includes a rotating rod, an extendable rod and an elastic member; wherein one end of the rotating rod is in rotatable connection with the contact portion, the other end of the rotating rod is in movable connection with the extendable rod; the elastic member is connected between the rotating rod and the extendable rod; the elastic member is configured to drive the extendable rod to extend out from the rotating rod along an axial direction of the rotating rod; when the guide assembly moves along the first path, the extendable rod is configured to extend to join with the movable position-limiting member of the guide assembly, so as to drive the movable position-limiting member of the guide assembly to separate from a first butt joint hole and a second butt joint hole.

In an embodiment, after the movement mechanism is triggered, the effector assembly moves away from the movement mechanism along the first path, and drives the movement mechanism to move to a position before being triggered, so as to be triggered again.

In an embodiment, the movement mechanism is in rotatable connection with the frame body via a rotation-shaft member, wherein a torsion spring is provided at the rotation-shaft member; wherein an elastic restoring force of the torsion spring is configured to drive the movement mechanism to rotate to the position before being triggered.

In an embodiment, the frame body is provided with a guide structure; wherein the guide structure contacts with the effector assembly, so as to guide the effector assembly to move along the first path, while the disassembling tool disassembles the guide assembly.

In an embodiment, the frame body is provided with a position-limiting structure; wherein the position-limiting structure is configured to contact with the guide assembly in the separate state, so as to retain the guide assembly in the separate state inside the frame body, when the effector assembly moves away from the frame body along the first path.

In an embodiment, the frame body includes an accommodation cavity, wherein the movement mechanism is arranged inside the accommodation cavity; the frame body is provided with a first opening, through which the guide assembly enters into and exits from the accommodation cavity, so as to enable the guide assembly to move inside the accommodation cavity along the first path.

In an embodiment, the frame body is further provided with a second opening, through which the guide assembly is taken out from the frame body after the guide assembly is separated from the effector assembly.

In an embodiment, a surgical instrument is provided, including a handle assembly, a barrel assembly, an effector assembly and a guide assembly as described in any one of the above embodiments; wherein a proximal end of the barrel assembly is connected with the handle assembly, a distal end of the barrel assembly is connected with the effector assembly, wherein the effector assembly includes a first end of the effector assembly and a second end of the effector assembly, wherein the first end of the effector assembly is in movable connection with the second end of the effector assembly, the first end of the effector assembly and the second end of the effector assembly are capable of moving relative to each other to close and open; wherein the guide assembly is capable of joining with the first end of the effector assembly or the second end of the effector assembly.

In an embodiment, a surgical instrument is provided, including:
a handle assembly;
a barrel assembly, wherein a proximal end of the barrel assembly is connected with the handle assembly;
an effector assembly, wherein a distal end of the barrel assembly is connected with the effector assembly;
a guide assembly, which includes a main-body member and a movable position-limiting member; wherein the main-body member includes a joining portion and a guide portion, wherein the joining portion is joined with an end of the effector assembly of the surgical instrument, and the guide portion is arranged at the joining portion; the movable position-limiting member is in movable connection with the main-body member, wherein the movable position-limiting member is capable of moving relative to the main-body member, so as to switch the joining portion and the effector assembly between a separate state and a joined state;
a disassembling tool, which is configured to drive the guide assembly and the effector assembly of the surgical instrument to switch from the joined state to the separate state; and
a packaging assembly, which includes a first packaging member and a second packaging member, wherein the first packaging member is configured to package the handle assembly, the barrel assembly and the effector assembly, and the second packaging member is configured to package the guide assembly and the disassembling tool.

In an embodiment, the second packaging member is connected with an upper surface, a lower surface, a side portion or an inner portion, of the first packaging assembly.

In an embodiment, the second packaging member is connected with the first packaging assembly via a snap-in structure of interference fit.

In an embodiment, the guide assembly and the disassembling tool are arranged side by side or stacked inside the second packaging member.

In an embodiment, a guide assembly for a surgical instrument is provided, including:
a main-body member, which includes a joining portion and a guide portion, wherein the joining portion is joined with an end of an effector assembly of the surgical instrument, and the guide portion is arranged at the joining portion;
a cross-section area of the guide portion gradually decreases from a proximal end of the guide portion to a distal end of the guide portion; a central axis of the guide portion along a length direction and a central axis of the effector assembly along the length direction form an angle a; wherein a value range of the angle a is: 5°≤a≤45°; and
a movable position-limiting member, which is in movable connection with the joining portion or the guide portion, wherein the movable position-limiting member is capable of moving relative to the main-body member, so as to switch the joining portion and the end of the effector assembly between a separate state and a joined state; wherein in the joined state, the movable position-limiting member is snap-fitted with the end of the effector assembly, so as to form a smooth surface to contact with tissue.

In an embodiment, the movable position-limiting member is provided with snap-fit portion(s), the main-body member and/or the effector assembly are/is provided with a snap-fit structure, which is snap-fitted with the snap-fit portion, wherein the snap-fit portion(s) is(are) snap-fitted with the snap-fit structure(s) in the joined state.

In an embodiment, the snap-fit portion is configured as a snap-fit column; a movement plane of the snap-fit column is parallel to an axis of the main-body member; wherein the joining portion is provided with a first butt joint hole, which perforates through the joining portion and configured to align with a second butt joint hole at the end of the effector assembly of the surgical instrument; wherein in the joined state, the snap-fit column penetrates through the first butt joint hole and the second butt joint hole, so as to join the joining portion with the end of the effector assembly.

According to a surgical instrument, a guide assembly and a disassembling tool thereof in an above-mentioned embodiment, the guide assembly includes a main-body member and a movable position-limiting member. The main-body member includes a joining portion and a guide portion. The joining portion is joined with an end of an effector assembly of the surgical instrument, and the guide portion is arranged at the joining portion. The movable position-limiting member is in movable connection with the joining portion or the guide portion, and the movable position-limiting member is capable of moving relative to the main-body member, so as to switch the joining portion and the end of the effector assembly between a separate state and a joined state. When the movable position-limiting member is subjected to a force along a first direction, the joining portion and the end of the effector assembly are switched from the joined state to the separate state; wherein the first direction is different from a direction of a reaction force, which force is applied by a working object to the end of the effector assembly during working. When it is necessary to operate on tissue in a relatively narrow space, the operator can join the joining portion of the guide assembly with the end of the effector assembly of the surgical instrument, so as to guide the effector assembly into the tissue in the narrow space through the guide portion of the guide assembly. When the guide assembly is not needed, the operator can operate the movable position-limiting member to move, so as to switch the guide assembly and the surgical instrument from a joined state to a separate state, and then disassemble the guide assembly. On the one hand, the operator is capable of satisfying a surgical requirement with only one surgical instrument with a guide assembly, without having to operate another instrument, which is conducive to reducing medical costs and expenses. On the other hand, since a direction of a reaction force, which force is applied by a working object to the end of the effector assembly during working, is different from the first direction, when the effector assembly is used to clamp tissue, the joining portion and the end of the effector assembly are not easy to switch from the joined state to the separate state under the reaction force of the tissue, which is beneficial to prevent the guide assembly from falling from the end of the effector assembly during operation and reduce a risk of using the surgical instrument

### BRIEF DESCRIPTION OF THE DRAWINGS

FIG. 1 is a structural diagram for a surgical instrument in an embodiment.
FIG. 2 is a structural diagram for a guide assembly in an embodiment.
FIG. 3 is a side view for a guide assembly in an embodiment.
FIG. 4 is a top view for a guide assembly in an embodiment.
FIG. 5 is a structural diagram for a main main-body member in an embodiment.
FIG. 6 is a side view for a main-body member in an embodiment.
FIG. 7 is a structural diagram for a movable position-limiting member in an embodiment.
FIG. 8 is a side view for a movable position-limiting member in an embodiment.
FIG. 9 is a structural diagram for a first effector assembly in an embodiment.
FIG. 10 is a structural diagram for an end of a first effector assembly in an embodiment.
FIG. 11 is a structural diagram for a guide assembly and an effector assembly in a separate state in an embodiment.
FIG. 12 is a structural diagram for a guide assembly and an end of an effector assembly in a slidably joined state in an embodiment.
FIG. 13 is a structural diagram for a guide assembly and an end of the effector assembly in a joined state in an embodiment of this disclosure.
FIG. 14 is a top view for a guide assembly and an end of the effector assembly in a joined state in an embodiment.
FIG. 15 is a structural diagram for a guide assembly and an end of the effector assembly in a reversely joined state in an embodiment.
FIG. 16 is an enlarged view for a portion C in FIG. 15.
FIG. 17 is a structural diagram for a disassembling tool in an embodiment.
FIG. 18 is a cross-section view for a disassembling tool in an embodiment.
FIG. 19 is a structural diagram for a separating member in an embodiment.
FIG. 20 is a structural diagram for an effector assembly, which is joined with a guide assembly, while being assembled and dissembled by a disassembling tool in an embodiment.
FIG. 21 is a structural diagram for an effector assembly, which is joined with a guide assembly, while being assembled and dissembled by a disassembling tool in an embodiment.
FIG. 22 is a structural diagram for an effector assembly, which is joined with a guide assembly, while being assembled and dissembled by a disassembling tool in an embodiment.
FIG. 23 is a cross-section view for an effector assembly, which is joined with a guide assembly, while being assembled and dissembled by a disassembling tool in an embodiment.
FIG. 24 is a cross-section view for an effector assembly, which is joined with a guide assembly, while being assembled and dissembled by a disassembling tool in an embodiment.
FIG. 25 is a cross-section view for an effector assembly, which is joined with a guide assembly, while being assembled and dissembled by a disassembling tool in an embodiment.
FIG. 26 is a cross-section view for an effector assembly, which is joined with a guide assembly, while being assembled and dissembled by a disassembling tool in an embodiment.
FIG. 27 is a partially enlarged view for FIG. 26.
FIG. 28 is a cross-section view for a separating member which is inserted into a knife slot in an embodiment.
FIG. 29 is a structural diagram for a first packaging member in an embodiment.
FIG. 30 is a structural diagram for a second packaging member in an embodiment.
FIG. 31 is a schematic diagram for an inner structure of a second packaging member in an embodiment.
FIG. 32 is a structural diagram for an inner liner in an embodiment.
   Reference numbers in FIGS.1-32 are as follows.
   100-handle assembly, 110-fixed handle, 120-trigger;
   200-barrel assembly;
   300-effector assembly, 310-first effector assembly, 311-plug-in slot, 312-snap-fit structure, 313-slidable guide portion, 314-knife slot, 320-second effector assembly;
   400-guide assembly, 410-main-body member, 411-joining portion, 412-guide portion, 413-first position-limiting portion, 4131-second guide surface, 414-second position-limiting portion, 415-guide channel, 416-snap-fit opening, 420-movable position-limiting member, 421-slidable snap-fit portion, 4211-first guide surface, 4212-movement-stopping surface, 422-abutting portion, 420a-first cantilever, 420b-second cantilever, 420c-connection arm;
   500-disassembling tool, 510-frame body, 511-third position-limiting portion, 512-fifth position-limiting portion, 513-accommodation cavity, 514-first opening, 515-second opening, 520-movement mechanism, 521-separating member, 5211-hook-shaped structure, 5212-fourth position-limiting portion, 522-powering member, 600-packaging assembly, 610-first packaging member, 620-second packaging member, 621-outer housing, 6211-placing step, 622-inner liner, 6221-first assembling groove, 6222-second assembling groove, 623-structure for taking and placing, 630-snap-in structure.

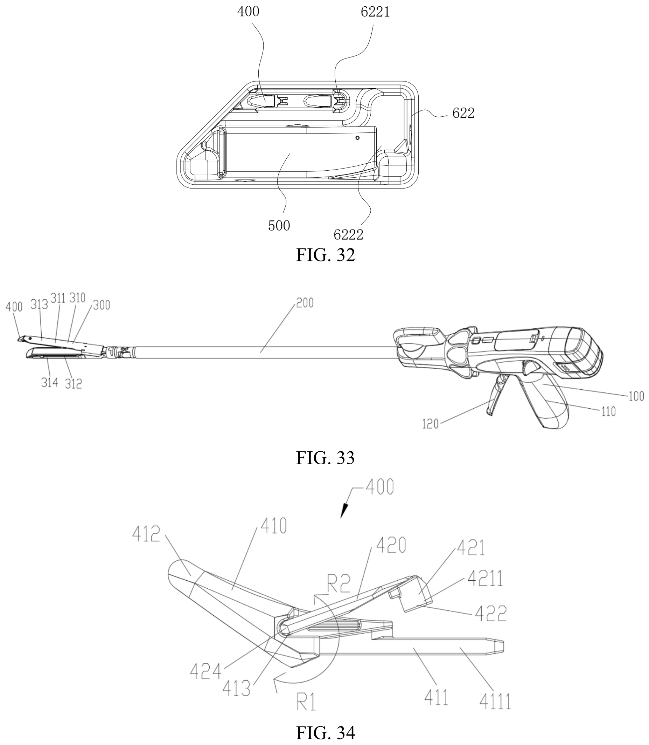

FIG. 33 is a structural diagram for a surgical instrument, when a guide assembly is joined with an effector assembly in an embodiment of this disclosure.
FIG. 34 is a structural diagram for a guide assembly from a side view in an embodiment of this disclosure.

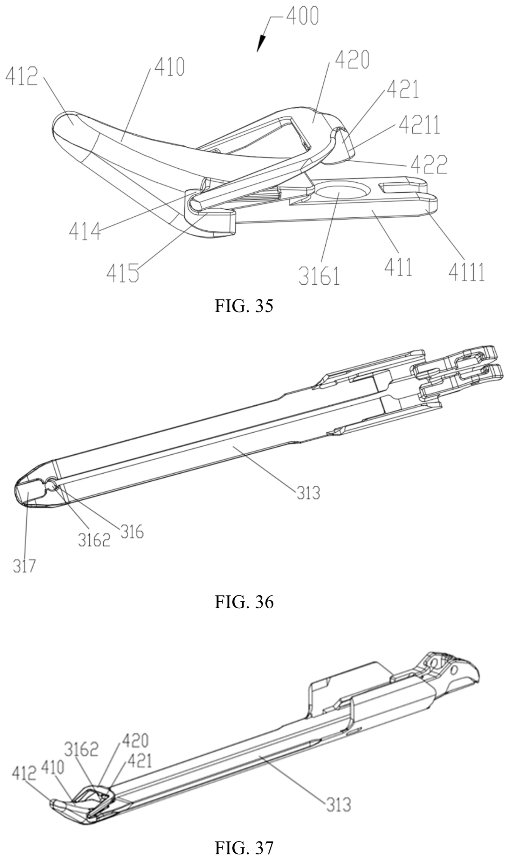

FIG. 35 is a structural diagram for a guide assembly in a three-dimensional perspective in an embodiment of this disclosure.
FIG. 36 is a structural diagram for a staple anvil in an embodiment of this disclosure.
FIG. 37 is a diagram showing fitting between a guide assembly and a staple anvil in a separate state in an embodiment of this disclosure.

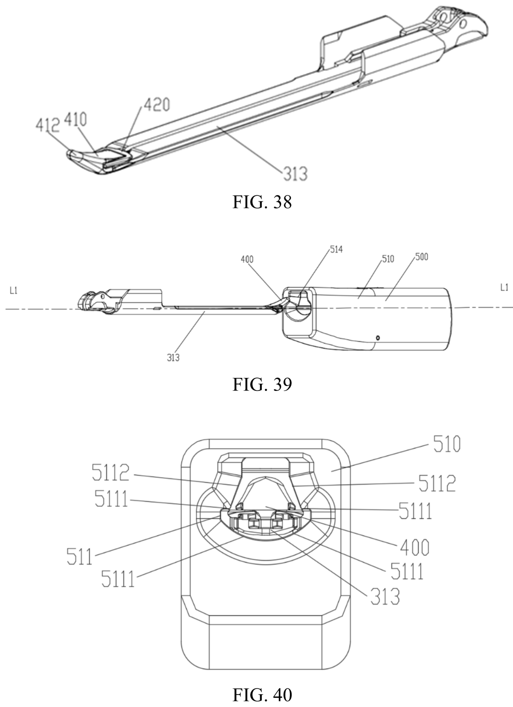

FIG. 38 is a diagram showing fitting between a guide assembly and a staple anvil in a joined state in an embodiment of this disclosure.
FIG. 39 is a structural diagram for a staple anvil and a guide assembly, which move along a first path toward a disassembling tool, in an embodiment of this disclosure.
FIG. 40 is a diagram showing fitting between a guide structure of a disassembling tool, a staple anvil and a guide assembly in an embodiment of this disclosure.

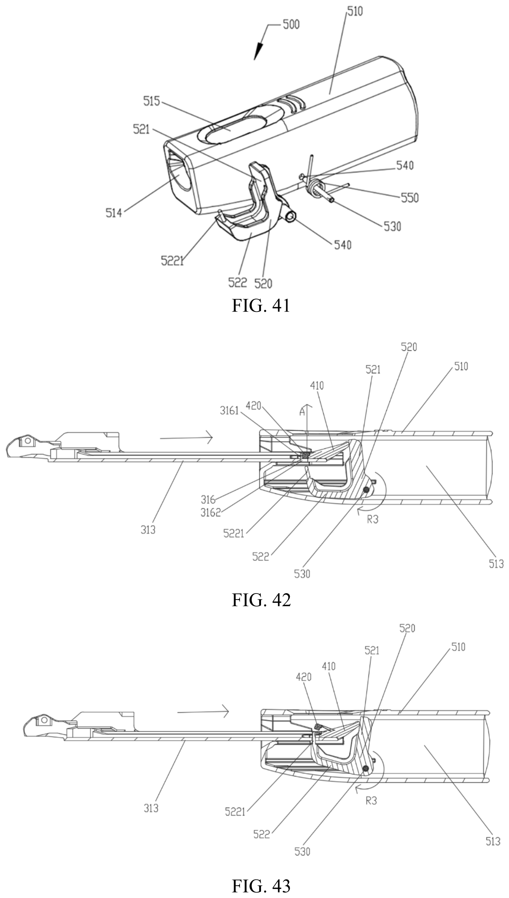

FIG. 41 is an exploded view for a disassembling tool in an embodiment of this disclosure.
FIG. 42 is a structural diagram, in which the guide assembly contacts with the movement mechanism in an embodiment of this disclosure.
FIG. 43 is a structural diagram, in which a guide assembly drives a movement mechanism to rotate in an embodiment of this disclosure.

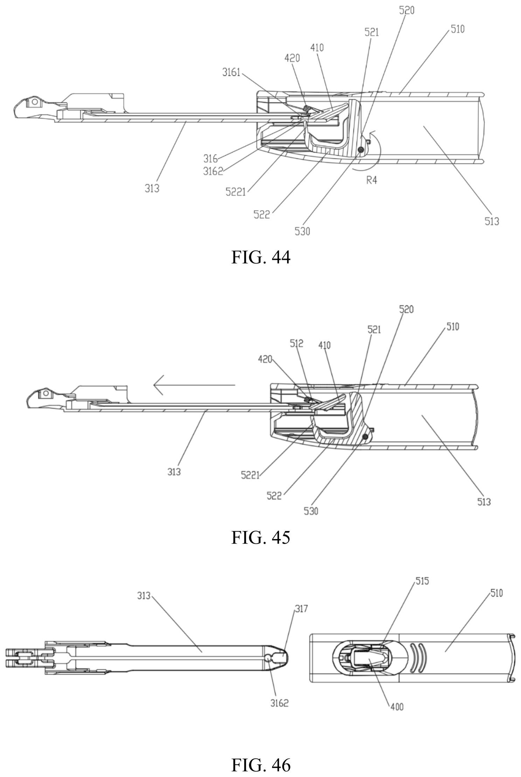

FIG. 44 is a structural diagram for automatic resetting of a movement mechanism in an embodiment of this disclosure.
FIG. 45 is a structural diagram, in which a guide assembly and a staple anvil are separated from a disassembling tool in an embodiment of this disclosure.
FIG. 46 is a structural diagram, in which a staple anvil is separated from a disassembling tool and a guide assembly remains inside the disassembling tool, in an embodiment of this disclosure.

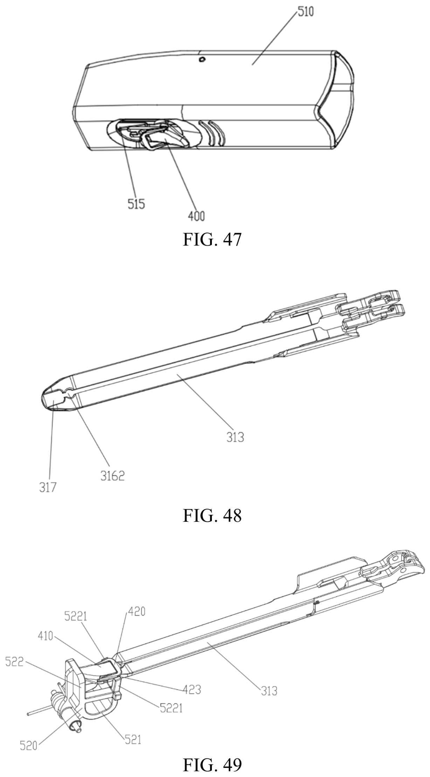

FIG. 47 is a schematic diagram for pouring out a guide assembly from a second opening of a disassembling tool in an embodiment of this disclosure.
FIG. 48 is a structural diagram for a staple anvil in a second embodiment of this disclosure.
FIG. 49 is a structural diagram, in which a movement mechanism and a guide assembly contacts with each other, in a third embodiment of this disclosure.

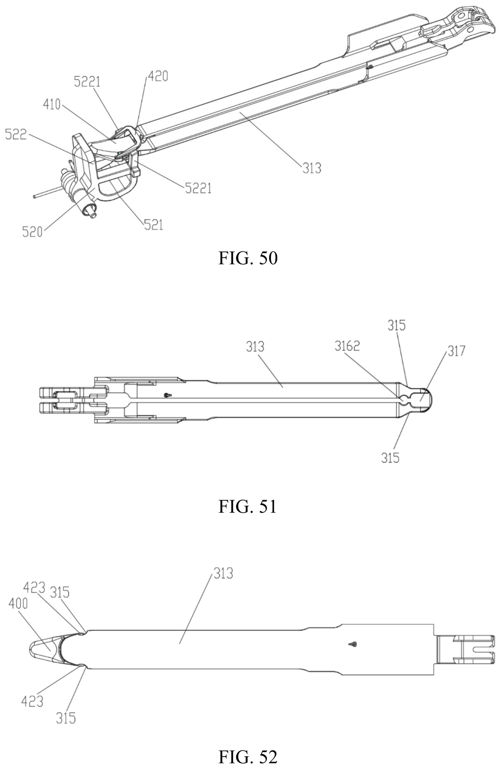

FIG. 50 is a schematic diagram, in which a movement mechanism pushes a guide assembly to switch from a joined state to a separate state, in a third embodiment of this disclosure.
FIG. 51 is a structural diagram for a staple anvil in a third embodiment of this disclosure.
FIG. 52 is a structural diagram for a staple anvil in a fourth embodiment of this disclosure.

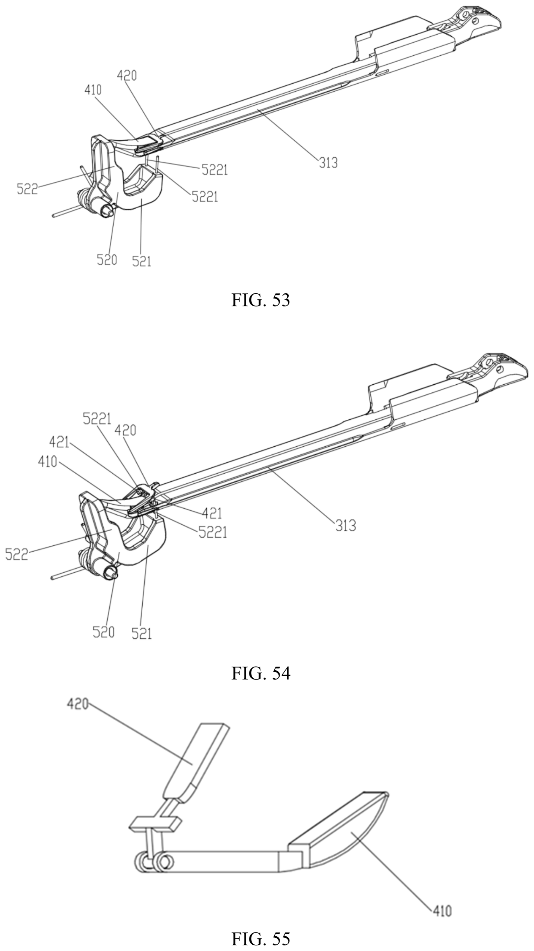

FIG. 53 is a structural diagram for a movement mechanism and a guide assembly in a fifth embodiment of this disclosure.
FIG. 54 is a diagram, in which a movement mechanism pushes a guide assembly to switch from a joined state to a separate state, in a fifth embodiment of this disclosure.
FIG. 55 is a structural diagram for a guide assembly in a sixth embodiment of this disclosure.

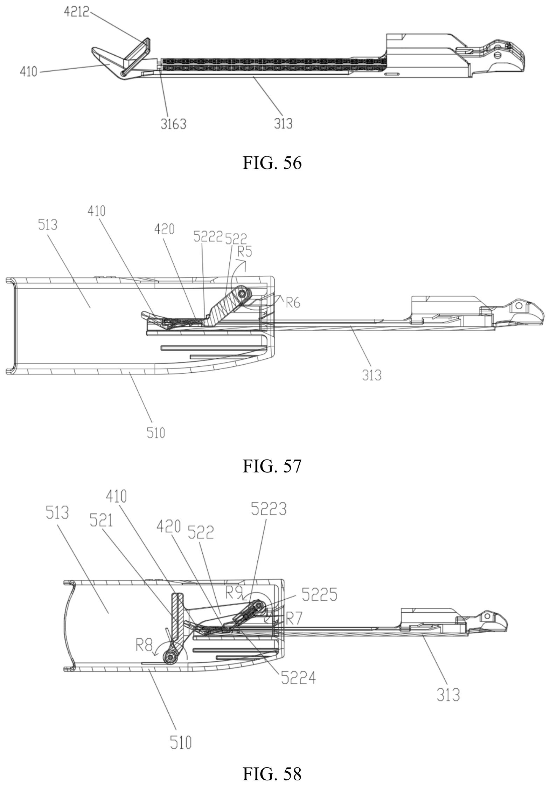

FIG. 56 is a structural diagram for a guide assembly and a staple anvil in a seventh embodiment of this disclosure.
FIG. 57 is a structural diagram, in which a guide assembly and a staple anvil extend into a disassembling tool, in an eighth embodiment of this disclosure.
FIG. 58 is a structural diagram, in which a guide assembly and a staple anvil extend into a disassembling tool, in a ninth embodiment of this disclosure.

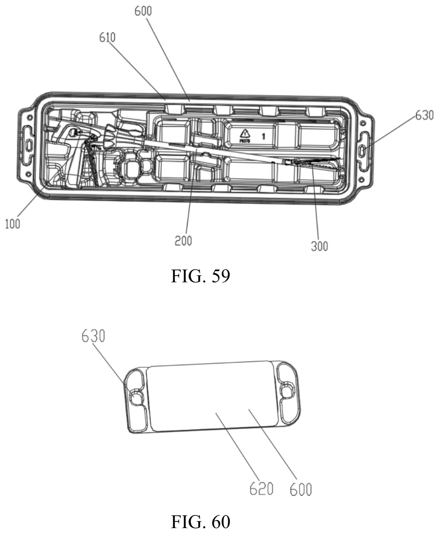

FIG. 59 is a structural diagram for a first packaging member for packaging a surgical instrument in an embodiment of this disclosure.
FIG. 60 is a structural diagram for a second packaging member in an embodiment of this disclosure.

Reference numbers in FIGS. 33-60 are as follows.

100, handle assembly; 110, fixed handle; 120, trigger; 200, barrel assembly; 300, effector assembly; 310, end of the effector assembly; 311, first end of the effector assembly; 312, second end of the effector assembly; 313, staple anvil; 314, staple cartridge base; 315, recessed portion; 316, snap-fit structure; 3161, first butt joint hole; 3162, second butt joint hole; 3163, snap-fit groove; 317, plug-in groove; 400, guide assembly; 410, main-body member; 411, joining portion; 4111, plug-in structure; 412, guide portion; 413, rotation-shaft hole; 414, first position-limiting portion; 415, second position-limiting portion; 420, movable position-limiting member; 421, snap-fit portion; 4211, snap-fit column; 4212, snap-fit rod; 422, assembling portion; 423, disassembling portion; 424, rotation-shaft portion; 500, disassembling tool; 510, frame body; 511, guide structure; 5111, upper-and-lower guide structure; 5112, left-and-right guide structure; 512, position-limiting structure; 513, accommodation cavity; 514, first opening; 515, second opening; 520, movement mechanism; 521, contact portion; 522, effector portion; 5221, pushing shaft; 5222, hook member; 5223, rotating rod; 5224, extendable rod; 5225, elastic member; 530, rotation-shaft member; 540, rotation-shaft hole; 550, torsion spring; 600, packaging assembly; 610, first packaging member; 620, second packaging member; 630, snap-in structure.

### DESCRIPTION OF THE PREFERRED EMBODIMENTS

This disclosure is further described in detail below through specific embodiments in combination with the accompanying drawings. In different embodiments, similar elements adopt associated similar reference numbers. In the following embodiments, many details are described so as to make this disclosure better understood. However, one skilled in the art can easily recognize that some of the features can be omitted in different cases, or can be replaced by other elements, materials and methods. In some cases, some operations related to this disclosure are not shown or described in the description, so as to avoid a core part of this disclosure being inundated by too many descriptions. For one skilled in the art, it is not necessary to describe these related operations in detail, as they can fully understand the relevant operations according to the description in this disclosure and the general technical knowledge in the art.

In addition, the features, operations, or characteristics described in this disclosure may be combined in any appropriate manner to form various embodiments. At the same time, the steps or actions in the method description can also be exchanged or regulated in order in a manner obvious to one skilled in the art. Therefore, the various sequences in the description and the drawings are only for the purpose of clearly describing a certain embodiment, and do not mean that they are necessary sequences, unless it is otherwise specified that one of the sequences must be followed.

The terms "first", "second", etc., in the description are used to distinguish different objects, rather than to describe a specific order. In addition, the terms "connection", and "linkage" mentioned in this disclosure include direct and indirect connection (linkage), unless otherwise specified. In this embodiment, "end" or "distal end" refers to an end of a surgical instrument, which end is away from an operation, when operating the surgical instrument, that is, an end which is adjacent to a patient, while "proximal end" refers to an end, which is adjacent an operation, when operating the surgical instrument, that is, an end which is away from a patient; "axial direction" refers to a direction of a line which connects a center of the end and a center of the proximal end, and "radial direction" refers to a direction which is perpendicular to the axial direction.

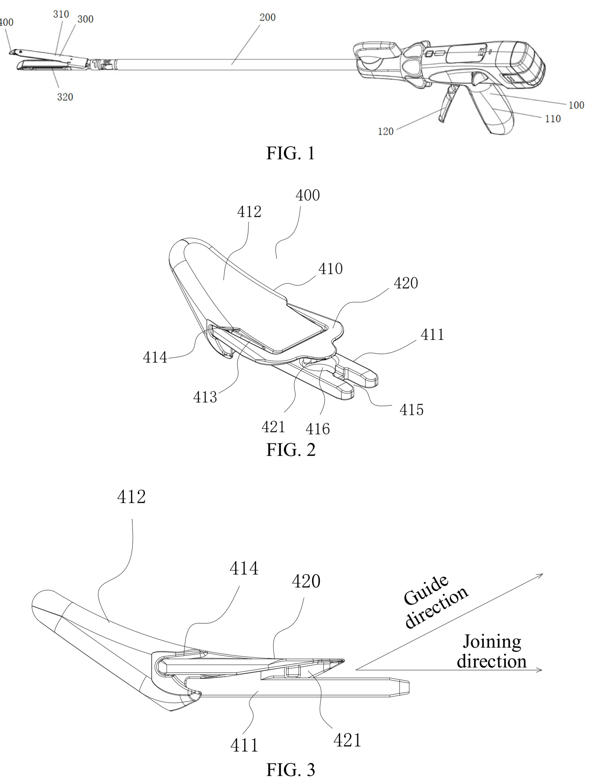

A surgical stapling instrument is a surgical instrument which is commonly used in minimally invasive surgery. Please refer to FIG. 1. The surgical stapling instrument generally includes a handle assembly 100, a barrel assembly 200 and an effector assembly 300. The barrel assembly 200 includes a closed transmission chain and a firing transmission chain. The handle assembly 100 includes a fixed handle 110 and at least one trigger 120.

When operating the surgical stapling instrument, an operator can hold the fixed handle 110 and insert the effector assembly 300 into a lesion site in a human body through a slender barrel assembly 200. The trigger 120 then triggers the closed transmission chain to close jaws of the effector assembly 300 for clamping a specific position, which is adjacent to a lesion site. Then, the trigger 120 triggers the firing transmission chain, so as to enable a cutting knife inside the effector assembly 300 to cut tissue, and enable stitching staples inside the effector assembly 300 to stitch the tissue, while cutting. In other embodiments, closing and firing operations may have other schemes, such as the two triggers 120 being operated separately to perform closing and firing. Alternatively, an electric closing and firing solution can be employed. Specifically, the closed transmission chain and the firing transmission chain can be driven by a motor or manually.

Please refer to FIGS. 1 to 14. In an embodiment, a surgical instrument is provided. The surgical instrument includes a handle assembly 100, a barrel assembly 200, an effector assembly 300 and a guide assembly 400.

A proximal end of the barrel assembly 200 is connected with the handle assembly 100, and an end of the barrel assembly 200 is connected with the effector assembly 300. The effector assembly 300 includes a first effector assembly 310 and a second effector assembly 320. The first effector assembly 310 and the second effector assembly 320 are in movable connection with each other, an end of the first effector assembly 310 and an end of the second effector assembly 320 is capable of moving relative to each other, so as to close and open. The guide assembly 400 may be joined with the end of the first effector assembly 310 or the end of the second effector assembly 320.

When it is necessary to operate on tissue in a relatively narrow space, the operator can join the guide assembly 400 with the end of the first effector assembly 310 or the end of the second effector assembly 320 of the surgical instrument. The guide assembly 400 is an eagle-beak-shaped tip structure, so as to guide the effector assembly 300 into tissue in the narrow space through the guide assembly 400. The operator is capable of satisfying a surgical requirement with only one surgical instrument with a guide assembly 400, without having to operate another instrument, which is conducive to reducing medical costs and expenses.

In an embodiment, a guide assembly 400 for a surgical instrument is provided. The guide assembly 400 is detachably joined with the effector assembly 300 in the above embodiment for use.

The guide assembly 400 of this embodiment includes a main-body member 410 and a movable position-limiting member 420. The main-body member 410 includes a joining portion 411 and a guide portion 412. The joining portion 411 is joined with an end of the effector assembly 300 of the surgical instrument, and the guide portion 412 is arranged at the joining portion 411. The movable position-limiting member 420 is in movable connection with the joining portion 411 or the guide portion 412, and the movable position-limiting member 420 is capable of moving relative to the main-body member 410, so as to switch the joining portion 411 and the end of the effector assembly 300 between a separate state and a joined state.

The movable position-limiting member 420 includes a slidable snap-fit portion 421, which is slidably arranged relative to the slidable guide portion 313 at the end of the effector assembly 300, and the slidable snap-fit portion 421 is capable of sliding along the slidable guide portion 313. The slidable snap-fit portion 421 is fitted with the snap-fit structure 312 at the end of the effector assembly 300, and the slidable snap-fit portion 421 is capable of sliding to snap-fit with the snap-fit structure 312.

When the joining portion 411 is joined with the end of the effector assembly 300, the movable position-limiting member 420 is subjected to a force along a joining direction, the slidable snap-fit portion 421 is in slidable contact with the slidable guide portion 313 at the end of the effector assembly 300, and the slidable snap-fit portion 421 slides along a guide direction of the slidable guide portion 313. While the slidable snap-fit portion 421 slides, the movable position-limiting member 420 generates a non-flexible deformation when abutting against the slidable guide portion 313, and releases the non-flexible deformation when the slidable snap-fit portion 421 slides to a joining position, so as to snap-fit with the snap-fit structure 312 of the effector assembly 300, wherein the guide direction of the slidable guide portion 313 intersects with a joining direction of the effector assembly 300.

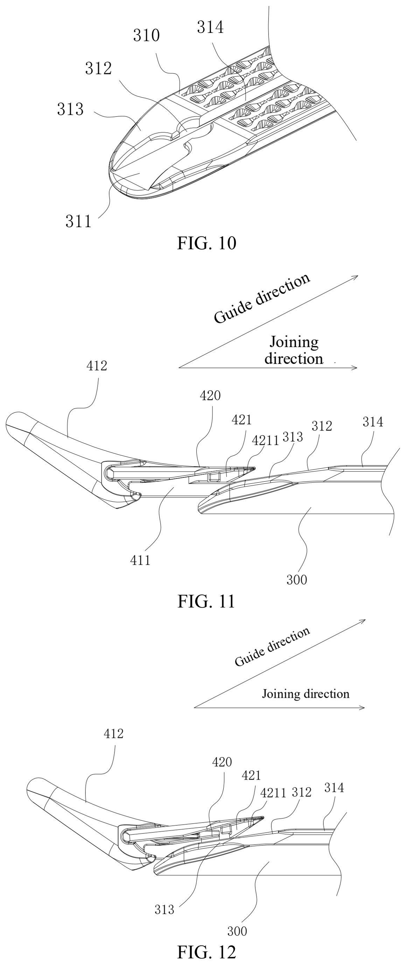

As shown in FIG. 3 and FIG. 11, the joining direction is an axial direction of the effector assembly 300, i.e., a length direction of the effector assembly 300, and the guide direction is a sliding direction of the movable position-limiting member 420, and the guide direction is inclined relative to the joining direction.

The main-body member 410 may be an integrated member made of a hard material, such as metal, while the joining portion 411 and the guide portion 412 may be integrated. The integrated main-body member 410 has a higher structural stability, and is capable of reducing a gap at a joint between portions, making it less likely to have residual tissue and liquid. The joining portion 411 and the guide portion 412 are also two portions made of a hard material, such as metal. The joining portion 411 and the guide portion 412 can be fixed together by means of screw connection, snap-fit or welding. The guide portion 412 is similar to an eagle-beak-shaped structure, and is a tilted tip structure as whole, which is capable of guiding to insert into a narrow space. Of course, a tip of the guide portion 412 is provided with a non-sharp structure, such as a transition arc to avoid puncturing tissue.

The effector assembly 300 includes a first effector assembly 310 and a second effector assembly 320. The first effector assembly 310 is a staple anvil, and the second effector assembly 320 is a staple cartridge base. In this embodiment, the guide assembly 400 is joined with the first effector assembly 310, and the guide assembly 400 is arranged at the end of the first effector assembly 310. Of course, the guide assembly 400 can also be joined with the second effector assembly 320, and the guide assembly 400 can be arranged at the end of the second effector assembly 320; and both arrangements can guide the effector assembly 300 into a narrow space.

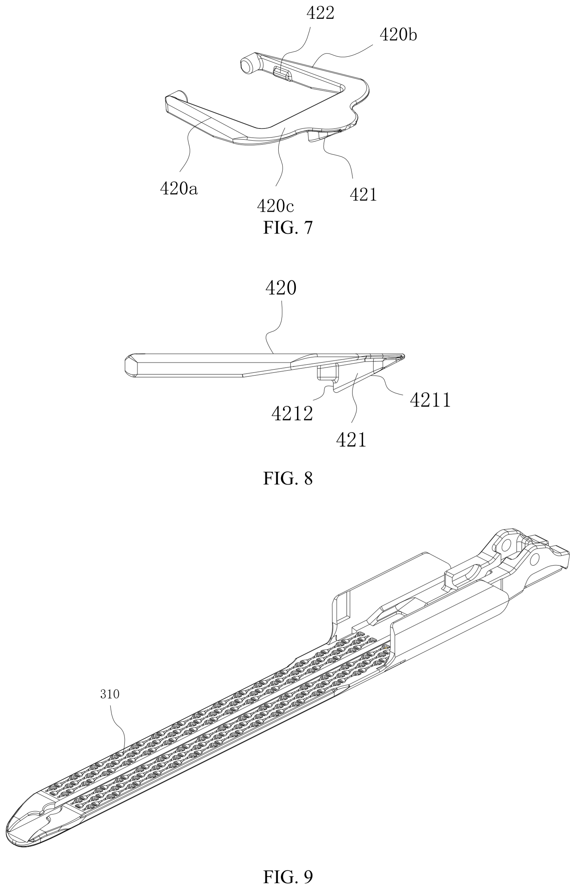

Please refer to FIGS. 9 and 10. The first effector assembly 310 can be provided with a plug-in slot 311, a snap-fit structure 312 and a slidable guide portion 313. The first effector assembly 310 also includes a knife slot 314. The plug-in slot 311 is located at an end of the first effector assembly 310. The plug-in slot 311, the snap-fit structure 312 and the knife slot 314 are connected in sequence along the joining direction. The slidable guide portion 313 extends from the plug-in slot 311 to the snap-fit structure 312.

Please refer to FIGS. 11 to 14. When the guide assembly 400 is joined with the first effector assembly 310, the guide assembly 400 is joined with the end of the first effector assembly 310 along the joining direction, and the joining portion 411 is inserted into the plug-in slot 311. After the guide assembly 400 generates a pushing force along the joining direction, the slidable snap-fit portion 421 of the guide assembly 400 is in slidable contact with the slidable guide portion 313, and the slidable snap-fit portion 421 slides along the guide direction of the slidable guide portion 313; while the slidable snap-fit portion 421 slides, the movable position-limiting member 420 generates a non-flexible deformation when abutting against the slidable guide portion 313, and releases the non-flexible deformation when the slidable snap-fit portion 421 slide to the joining position, so as to snap-fit with the snap-fit structure 312 of the effector assembly 300, wherein the guide direction of the slidable guide portion 313 intersects with the joining direction of the effector assembly 300.

When it is necessary to operate on tissue in a relatively narrow space, the operator can join the joining portion 411 of the guide assembly 400 with the end of the effector assembly 300 of the surgical instrument, so as to guide the effector assembly 300 into the tissue in the narrow space through the guide portion 412 of the guide assembly 400. When the guide assembly 400 is not needed, the operator can use a disassembling tool to move the movable position-limiting member 420 to move, so as to switch the guide assembly 400 and the surgical instrument from the joined state to the separate state, and then disassemble the guide assembly 400.

The operator is capable of satisfying a surgical requirement with only one surgical instrument with a guide assembly 400, without having to operate another instrument, which is conducive to reducing medical costs and expenses. Furthermore, since the slidable snap-fit portion 421 slides into and be snap-fitted with the snap-fit structure 312 of the effector assembly 300 through deformation, when the end of the effector assembly 300 operates, it is necessary to apply a deformation force to the slidable snap-fit portion 421, so as to switch from the joined state to the separate state, which is beneficial to prevent the guide assembly 400 from falling from the end of the effector assembly 300 during operation and reduce a risk of using the surgical instrument.

Please refer to FIGS. 2, 7 and 8. In an embodiment, the movable position-limiting member 420 includes a first end and a second end, which ends are opposite to each other. The first end of the movable position-limiting member 420 is in movable connection with the main-body member 410. The movable position-limiting member 420 can be in movable connection with the joining portion 411. The movable position-limiting member 420 can also be in movable connection with the guide portion 412. Alternatively, the movable position-limiting member 420 can also be in movable connection with a joint between the joining portion 411 and the guide portion 412. Both of these can realize the movable connection of the movable position-limiting member 420 relative to the main-body member 410.

The second end of the movable position-limiting member 420 forms a free end for snap-fit, and the slidable snap-fit portion 421 is arranged at the second end of the movable position-limiting member 420. The movable position-limiting member 420 is provided with an outer surface, which back-faces the joining portion 411, and an inner surface which faces the joining portion 411. The slidable snap-fit portion 421 protrudes from the inner surface of the movable position-limiting member 420, which inner surface faces the main-body member 410, so as to enable the movable position-limiting member 420 and the joining portion 411 to form a clamping structure for clamping the guide assembly 400 at the end of the effector assembly 300.

The slidable snap-fit portion 421 and the movable position-limiting member 420 may be an integrated structure, which has a higher structural stability. The slidable snap-fit portion 421 can also be fixed together by screw connection, snap-fit or welding.

The slidable snap-fit portion 421 may be a wedge-shaped structure, or may be a structure with another side being triangular. The slidable snap-fit portion 421 has a first guide surface 4211, which faces the joining portion 411 and extends to the end of the movable position-limiting member 420, such that when the movable position-limiting member 420 contacts with the slidable guide portion 313 at the end of the effector assembly 300, the first guide surface 4211 contacts with the slidable guide portion 313 to achieve a slidable and elastic snap-fit.

The slidable snap-fit portion 421 further has a movement-stopping surface 4212. The movement-stopping surface 4212 may be perpendicular to the joining direction, or nearly perpendicular to the joining direction. The movement-stopping surface 4212 and the first guide surface 4211 are arranged back-to-back. Due to the arrangement of the movement-stopping surface 4212, when the guide assembly 400 and the end of the effector assembly 300 are in the joined state, and the guide assembly 400 is subjected to an action force which drives the guide assembly 400 to move along an opposite direction of the joining direction, the movement-stopping surface 4212 can be snapped into the snap-fit structure 312, so as to prevent the guide assembly 400 from moving along an opposite direction of the joining direction, thereby avoiding abnormal separation of the guide assembly 400 and the end of the effector assembly 300. In other words, the slidable snap-fit portion 421 is a one-way slidable and elastic snap-fit structure, which can only be moved along the joining direction to achieve slidable and elastic snap-fit, and cannot be directly separated by a reverse movement.

Please refer to FIGS. 11 to 14. When the guide assembly 400 is joined with the end of the effector assembly 300, the first guide surface 4211 of the slidable snap-fit portion 421 slides inclinedly along the slidable guide portion 313, and the movable position-limiting member 420 gradually opens relative to the joining portion 411 and generates a non-flexible deformation. When the slidable snap-fit portion 421 slides to a joining position, the first guide surface 4211 of the slidable snap-fit portion 421 separates from the slidable guide portion 313, the movable position-limiting member 420 releases the non-flexible deformation, the movable position-limiting member 420 closes relative to the joining portion 411, and returns to its initial position, and the slidable snap-fit portion 421 is snap-fitted with the snap-fit structure 312 at the end of the effector assembly 300.

The arrangement of the first guide surface 4211 allows the movable position-limiting member 420 to be driven to move, so as to generate a non-flexible deformation and release the non-flexible deformation during joining, that is, the movable position-limiting member 420 can be driven to open and close relative to the joining portion 411, so as to join with the end of the effector assembly 300. During joining, no other operations are required, and the joining can be achieved by a slidable and elastic snap-fit in one step, which is simple and efficient.

Wherein, the sliding guide portion 313 can be a third guide surface. In a joined and aligned state, the third guide surface is parallel to the first guide surface 4211, and the third guide surface and the first guide surface 4211 are parallel to the guide direction. The third guide surface can guide the first guide surface 4211 to slide along a planar direction of the third guide surface.

In other embodiments, one of the first guide surface 4211 and the third guide surface is a guide surface, and the other one is replaced by a guide protrusion. The guide protrusion can be a hemispherical or another arc structure. Fitting between the guide protrusion and the guide surface can also guide the movable position-limiting member 420 to slide along the joining direction, thereby realizing the slidable snap-fit of the movable position-limiting member 420.

Please refer to FIGS. 1 to 8. In an embodiment, a movable connection between the first end of the movable position-limiting member 420 and the main-body member 410 can be a rotatable connection. The movable position-limiting member 420 can be provided with a protruded rotation-shaft member, and the main-body member 410 can be provided with a corresponding movement groove. The movement groove can be an axial hole groove which corresponds to the rotation-shaft member. The rotation-shaft member and the movement groove are rotationally connected, so as to realize a rotatable connection between the movable position-limiting member 420 and the main-body member 410. The rotation-shaft member may also be arranged at the main-body member 410, and the movement groove may be arranged at one end of the movable position-limiting member 420, so as to realize the rotatable connection between the movable position-limiting member 420 and the main-body member 410.

The main-body member 410 is provided with a first position-limiting portion 413, which may be a protruded structure, which protrudes from a side surface of the main-body member 410. The first position-limiting portion 413 may be arranged at a side surface of the joining portion 411, the first position-limiting portion 413 may also be arranged at a side surface of the guide portion 412, or the first position-limiting portion 413 may also be arranged at a side surface of a joint between the joining portion 411 and the guide portion 412. The first position-limiting portion 413 is arranged at a movement path of the movable position-limiting member 420, and the first position-limiting portion 413 is configured to squeeze the movable position-limiting member 420 to deform the movable position-limiting member 420. When the slidable snap-fit portion 421 slides inclinedly along the guide direction, the movable position-limiting member 420 is driven to rotate until it contacts with the first position-limiting portion 413. When the movable position-limiting member 420 continues to rotate relative to the first position-limiting portion 413, the first position-limiting portion 413 squeezes the movable position-limiting member 420, so as to enable the movable position-limiting member 420 to undergo a non-flexible deformation. Wherein, after the movable position-limiting member 420 contacts with the first position-limiting portion 413, when the slidable snap-fit portion 421 does not slide inclinedly to the joining position along the guide direction, the movable position-limiting member 420 does not separate from the first position-limiting portion 413, which can prevent the movable position-limiting member 420 from releasing energy generated by the non-flexible deformation in advance. In this way, when the movable position-limiting member 420 slides to the joining position, the movable position-limiting member 420 releases the energy generated by the non-flexible deformation again.

The first position-limiting portion 413 is arranged to allow the movable position-limiting member 420 to achieve a non-flexible deformation during rotation, so as to store deformation energy, and then be automatically snap-fitted by releasing the deformation energy, thereby achieving a slidable and elastic snap-fit between the guide assembly 400 and the end of the effector assembly 300. The guide assembly 400 only needs to be inserted into the end of the effector assembly 300 along the joining direction, and the operation is simple and efficient.

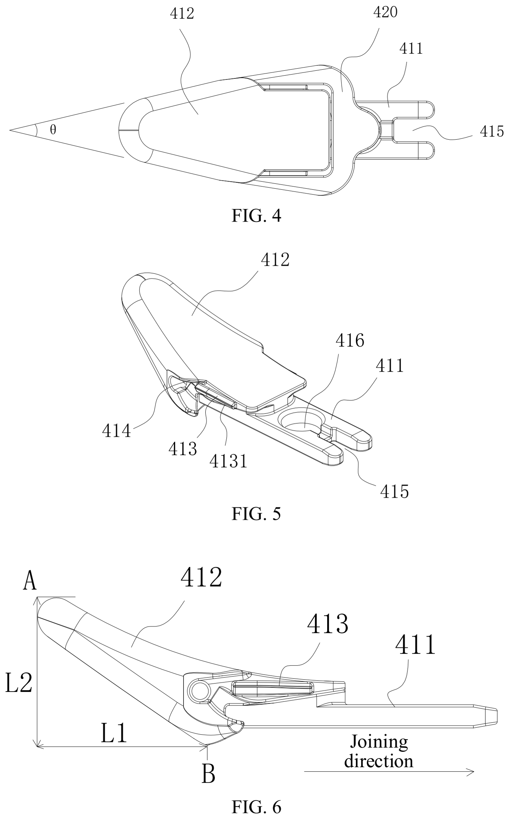

Please refer to FIGS. 5 to 7. In an embodiment, the first position-limiting portion 413 may be provided with a second guide surface 4131, which intersects with a rotation trajectory of the movable position-limiting member 420, and the second guide surface 4131 is not parallel to a plane on which the rotation trajectory of the movable position-limiting member 420 is located. After the sliding guide rotates, the movable position-limiting member 420 will abut against the second guide surface 4131 of the first position-limiting portion 413, and the second guide surface 4131 will squeeze the movable position-limiting member 420 to generate a non-flexible deformation.

A side surface of the movable position-limiting member 420 may be provided with a protruded abutting portion 422, which contacts with the second guide surface 4131 of the first position-limiting portion 413. The abutting portion 422 is a convex block structure with an arc surface, and the abutting portion 422 contacts with the second guide surface 4131 through the arc surface. The arrangement of the abutting portion 422 is capable of reducing a contact area between the movable position-limiting member 420 and the second guide surface 4131, which is more conducive to squeezing the movable position-limiting member 420 to achieve the non-flexible deformation.

In other embodiments, a second guide surface is provided at the movable position-limiting member 420 and a protruded abutting portion 422 is provided at the main-body member 410, so as to implement a position-limitation and squeeze the movable position-limiting member 420 for generating the non-flexible deformation.

Please refer to FIG. 1 to FIG. 8. In an embodiment, one first position-limiting portion 413 is respectively arranged on both sides of the main-body member 410. The two first position-limiting portions 413 are symmetrically arranged along a central cross-section of the main-body member 410.

The movable position-limiting member 420 can be a C-shaped double-cantilever structure, and the movable position-limiting member 420 includes a first cantilever 420a, a second cantilever 420b and a connection arm 420c. One end of the first cantilever 420a is in movable connection with one side of the main-body member 410, and the other end of the first cantilever 420a is connected with the connection arm 420c. One end of the second cantilever 420b is in movable connection with the other side of the main-body member 410, and the other end of the second cantilever 420b is connected with the connection arm 420c. The slidable snap-fit portion 421 is arranged at the connection arm 420c.

The first cantilever 420a, the second cantilever 420b and the connection arm 420c may be an integrated structure, which is more conducive to preserving energy generated by a non-flexible deformation and avoiding a loss of deformation energy due to activity at joint(s). The first cantilever 420a, the second cantilever 420b and the connection arm 420c can also be fixed together by welding, bonding, etc. This method can eliminate gap(s) at joint(s) and can also better preserve the energy generated by the non-flexible deformation.

The first position-limiting portion 413 on one side of the main-body member 410 is configured to squeeze the first cantilever 420a, and the first position-limiting portion 413 on the other side of the main-body member 410 is configured to squeeze the second cantilever 420b. The first position-limiting portions 413 on both sides of the main-body member 410 have two second guide surfaces which are inclinedly symmetrical. When the guide assembly 400 is joined with the end of the effector assembly 300, the first cantilever 420a and the second cantilever 420b rotate simultaneously to contact with the second guide surface of one first position-limiting portion 413. Under the guidance of the second guide surfaces of the two first position-limiting portions 413, the first cantilever 420a and the second cantilever 420b will be subjected to a non-flexible deformation, so as to expand outward to store energy, that is, the first cantilever 420a and the second cantilever 420b will deform in a direction away from each other, when the first cantilever 420a and the second cantilever 420b rotate. This deformation is a radial deformation along the joining direction.

The first cantilever 420a and the second cantilever 420b are both located outside the first position-limiting portion 413, which can prevent the first position-limiting portion 413 from directly contacting the tissue and preventing the first position-limiting portion 413 from scratching the tissue.

The movable position-limiting member 420 is configured as a C-shaped structure. During joining, the cantilevers on both sides expand outward to store energy. When the guide assembly 400 slides to the joining position, the cantilevers on both sides can simultaneously contract and release the stored energy to drive the slidable snap-fit portion 421 to be snap-fitted and joined with the snap-fit structure 312. The arrangement of the cantilevers on both sides can improve a stability of a deformation of the movable position-limiting member 420 and a stability of a snap-fit movement of the slidable snap-fit portion 421.

In other embodiments, the movable position-limiting member 420 may also include a cantilever, and a first position-limiting portion 413 is correspondingly provided at the main-body member 410. For example, the movable position-limiting member 420 is a single L-shaped cantilever structure, in which one cantilever is connected with one connection arm. When the single arm of the movable position-limiting member 420 is squeezed and contacted with the first position-limiting portion 413 on a side surface of the main-body member 410, a non-flexible deformation, which deformation expands outward to store energy, may be generated, thereby realizing a slidable and elastic snap-fit between the guide assembly 400 and the end of the effector assembly 300.

Please refer to FIG. 2 and FIG. 3. In an embodiment, the main-body member 410 is provided with a second position-limiting portion 414. The second position-limiting portion 414 is located on a movement trajectory of the movable position-limiting member 420. The second position-limiting portion 414 may be arranged at the joining portion 411, the second position-limiting portion 414 may also be arranged at the guide portion 412, or the second position-limiting portion 414 may also be arranged at the joining position between the joining portion 411 and the guide portion 412. The second position-limiting portion 414 is configured to limit a movement distance of the movable position-limiting member 420, that is, the second position-limiting portion 414 is configured to limit a maximum angle of the movable position-limiting member 420 relative to the joining portion 411.

The second position-limiting portion 414 can play a protective role and prevent the movable position-limiting member 420 from being separated from the first position-limiting portion 413 due to excessive rotation angle during slidable joining. The second position-limiting portion 414 can ensure that the movable position-limiting member 420 will not exceed the first position-limiting portion 413, thereby ensuring that the movable position-limiting member 420 can release the non-flexible deformation and join with the end of the effector assembly 300.

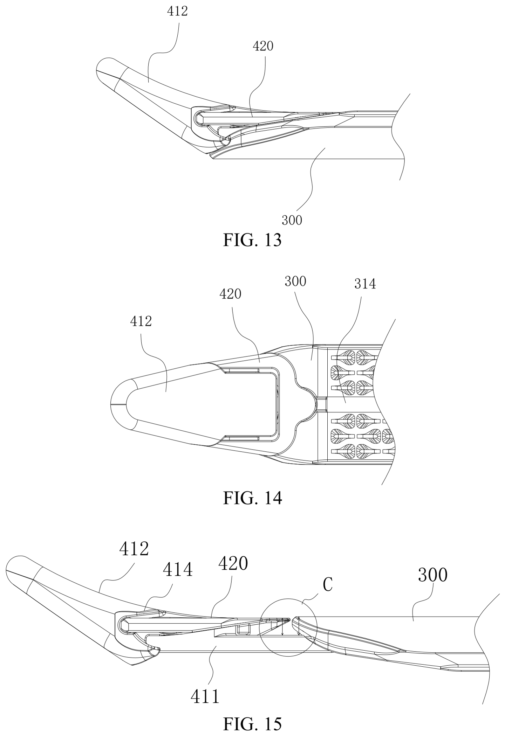

Please refer to FIG. 15. When the guide assembly 400 is rotated for 180° along the joining direction and then joined with the end of the effector assembly 300, that is, when the guide assembly 400 and the end of the effector assembly 300 are joined with each other in a reverse direction, due to the position-limiting of the second position-limiting portion 414, the movable position-limiting member 420 cannot be opened large enough for the end of the effector assembly 300 to be inserted into. Therefore, the second position-limiting portion 414 can also prevent the guide assembly 400 from being mistakenly assembled along the opposite direction.

In an embodiment, the second position-limiting portion 414 can be formed by extending from an edge of a movement groove of the main-body member 410, that is, the second position-limiting portion 414 and the main-body member 410 are an integrated structure, for example, the second position-limiting portion 414 can extend from a side surface of the guide portion 412. Such arrangement allows the second position-limiting portion 414 and the main-body member 410 to form a combined structure without connection trace(s), making a surface of the main-body member 410 smoother, facilitating a guidance of the guide assembly 400, and preventing the guide assembly 400 from forming a protruded structure that scratches tissue.

In other embodiments, the second position-limiting portion 414 may also be arranged at a rotation-shaft member, which is in rotatable connection with the movable position-limiting member 420 and the main-body member 410. By position-limiting for the rotation-shaft member, a maximum rotation distance of the movable position-limiting member 420 may be limited, thereby achieving a slidable and elastic joining of the movable position-limiting member 420 and avoiding incorrect assembly.

Please refer to FIGS. 1 to 6. In an embodiment, the guide portion 412 is an eagle-beak-shaped structure. The guide portion 412 has a first end and a second end, which ends are opposite to each other. The first end of the guide portion 412 is a tip end, and the guide portion 412 gradually increases from the first end to the second end. The guide portion 412 mainly includes a first side surface, a second side surface, a third side surface and a fourth side surface. The first side surface and the second side surface are inclined surfaces, which back-face each other, wherein the first side surface and the second side surface can also be curved surfaces, such as concave surfaces. The third side surface and the fourth side surface are side surfaces, which back-face each other, wherein the third side surface and the fourth side surface are connected with opposite sides of the first side surface and the second side surface. Side edges, where the first side surface, the second side surface, the third side surface and the fourth side surface are connected with each other, are provided with a chamfered structure, so as to make a contour of the guide portion 412 more rounded, which can avoid scratching of the tissue by sharp side edge(s). The third side surface and the fourth side surface are perpendicular to the joining direction.

A length L1 of the guide portion 412 is 6-9 cm, and a length direction of the guide portion 412 is parallel to the joining direction. As shown in FIG. 6, the length L1 of the guide portion 412 is a distance from point A to point B along a direction parallel to the joining direction. Point A is a vertex of the tip of the guide portion 412, and point B is a point on the guide portion 412, which point is farthest away from point A.

A ratio of the length L1 to a height L2 of the guide portion 412 is 0.8-2. The height of the guide portion 412 is perpendicular to the joining direction. As shown in FIG. 6, the height L2 of the guide portion 412 is a distance from point A to point B along a direction perpendicular to the joining direction.

Please refer to FIG. 4, an angle θ, which is formed by extended surfaces of two sides of the guide portion 412 intersecting, is 10-25°. That is, the angle θ, which is formed by extended surfaces of the third side and the fourth side of the guide portion 412 intersecting, is 10-25°.

The guide portion 412 adopts a structure of the above-mentioned proportional size, which can form a relatively sharp and upturned eagle-beak-shaped structure. The structure is small and three-dimensional, and can satisfy a surgical requirement with a sufficiently small size; and the guide portion 412 of this proportional size can also satisfy requirements of different usage scenarios.

Wherein, when the length L1 of the guide portion 412 is 7-8 cm, the ratio of the length L1 to the height L2 of the guide portion 412 is 1:1.5, and the angle θ, which is formed by the extended surfaces of two sides of the guide portion 412 intersecting, is 15-20°; a more compact and three-dimensional structure, which satisfies requirements of different usage scenarios, can be formed.

In an embodiment, the joining portion 411 is provided with a guide channel 415, and the joining portion 411 includes a first end and a second end, which ends are opposite to each other. The first end of the joining portion 411 is connected with the guide portion 412, and the second end of the joining portion 411 is provided with the guide channel 415. The guide channel 415 extends from an end surface of the second end of the joining portion 411 into an inner side of the joining portion 411 in the opposite direction of the joining direction.

When the guide assembly 400 is joined with the end of the effector assembly 300, the guide channel 415 is connected with the knife slot 314 of the effector assembly 300. The guide channel 415 is configured to avoid the cutting knife, so as to enable the cutting knife to move into the guide channel 415, when the cutting knife moves inside the knife slot 314. The joining portion 411 may be a C-shaped structure or an H-shaped structure, wherein one end of the C-shaped structure or the H-shaped structure may form two spaced-apart connection arms. The second end of the joining portion 411 is provided with two spaced-apart connection arms, and the two connection arms can be parallel arms arranged along the joining direction. A guide channel 415 is formed between the two connection arms of the joining portion 411, and the two connection arms of the joining portion 411 are inserted into the plug-in slot 311 of the effector assembly 300.

The guide channel 415 may be a guide groove with a size which corresponds with the knife slot 314, so as to enable the cutting knife to move into the guide groove. The arrangement of the guide channel 415 ensures that when the guide assembly 400 is joined with the end of the effector assembly 300, the joining portion 411 will not interfere with a movement of the cutting knife.

Please refer to FIG. 5. In an embodiment, the joining portion 411 is further provided with a snap-fit opening 416. The snap-fit opening 416 can be an avoidance structure which is perpendicular to the joining direction. The snap-fit opening 416 can be a blind hole at the joining portion 411, or a through hole at the joining portion 411. When the joining portion 411 is joined with the end of the effector assembly 300, a portion of the slidable snap-fit portion 421 penetrates through the snap-fit structure 312 and is located inside the snap-fit opening 416. The arrangement of the snap-fit opening 416 can prevent the slidable snap-fit portion 421 from contacting with the joining portion 411, thereby preventing the joining portion 411 from pushing up the movable position-limiting member 420.

In an embodiment, a guide assembly 400 for a surgical instrument is also provided.

Please refer to FIG. 1 to FIG. 14, the guide assembly 400 of this embodiment includes a main-body member 410 and a movable position-limiting member 420.

The main-body member 410 includes a joining portion 411 and a guide portion 412. The joining portion 411 is joined with an end of the effector assembly 300 of the surgical instrument, and the guide portion 412 is arranged at the joining portion 411. The movable position-limiting member 420 is in movable connection with the joining portion 411 or the guide portion 412, and the movable position-limiting member 420 is capable of moving relative to the main-body member 410, so as to switch the joining portion 411 and the end of the effector assembly 300 between a separate state and a joined state.

The main-body member 410 may be an integrated member made of a hard material, such as metal, while the joining portion 411 and the guide portion 412 may be integrated. The integrated main-body member 410 has a higher structural stability, and is capable of reducing a gap at a joint between portions, making it less likely to have residual tissue and liquid. The joining portion 411 and the guide portion 412 are also two portions made of a hard material, such as metal. The joining portion 411 and the guide portion 412 can be fixed together by means of screw connection, snap-fit or welding. The guide portion 412 is similar to an eagle-beak-shaped structure, and is a tilted tip structure as whole, which is capable of guiding to insert into a narrow space. Of course, a tip of the guide portion 412 is provided with a non-sharp structure, such as a transition arc to avoid puncturing tissue.

The effector assembly 300 includes a first effector assembly 310 and a second effector assembly 320. The first effector assembly 310 is a staple anvil, and the second effector assembly 320 is a staple cartridge base. In this embodiment, the guide assembly 400 is joined with the first effector assembly 310, and the guide assembly 400 is arranged at the end of the first effector assembly 310. Of course, the guide assembly 400 can also be joined with the second effector assembly 320, and the guide assembly 400 can be arranged at the end of the second effector assembly 320; and both arrangements can guide the effector assembly 300 into a narrow space.

The first effector assembly 310 can be provided with a plug-in slot 311, a snap-fit structure 312 and a slidable guide portion 313. The first effector assembly 310 also includes a knife slot 314. The plug-in slot 311 is located at an end of the first effector assembly 310. The plug-in slot 311, the snap-fit structure 312 and the knife slot 314 are connected in sequence along the joining direction. The slidable guide portion 313 extends from the plug-in slot 311 to the snap-fit structure 312.

The joining portion 411 is provided with a guide channel 415, and the joining portion 411 includes a first end and a second end, which ends are opposite to each other. The first end of the joining portion 411 is connected with the guide portion 412, and the second end of the joining portion 411 is provided with a guide channel 415. The guide channel 415 extends from an end surface of the second end of the joining portion 411 into an inner side of the joining portion 411 in the opposite direction of the joining direction.

When the guide assembly 400 is joined with the end of the effector assembly 300, the guide channel 415 is connected with the knife slot 314 of the effector assembly 300. The guide channel 415 is configured to avoid the cutting knife, so as to enable the cutting knife to move into the guide channel 415, when the cutting knife moves inside the knife slot 314.

Please refer to FIG. 5. In an embodiment, the joining portion 411 may be a C-shaped structure or an H-shaped structure, wherein one end of the C-shaped structure or the H-shaped structure may form two spaced-apart connection arms. The second end of the joining portion 411 is provided with two spaced-apart connection arms, and the two connection arms can be parallel arms arranged along the joining direction. A guide channel 415 is formed between the two connection arms of the joining portion 411, and the two connection arms of the joining portion 411 are inserted into the plug-in slot 311 of the effector assembly 300.

The guide channel 415 may be a guide groove with a size which corresponds with the knife slot 314, so as to enable the cutting knife to move into the guide groove. The arrangement of the guide channel 415 ensures that when the guide assembly 400 is joined with the end of the effector assembly 300, the joining portion 411 will not interfere with a movement of the cutting knife.

In an embodiment, a disassembling tool for a guide assembly for a surgical instrument is provided, wherein the disassembling tool is configured to separate and disassemble the guide assembly from the effector assembly.

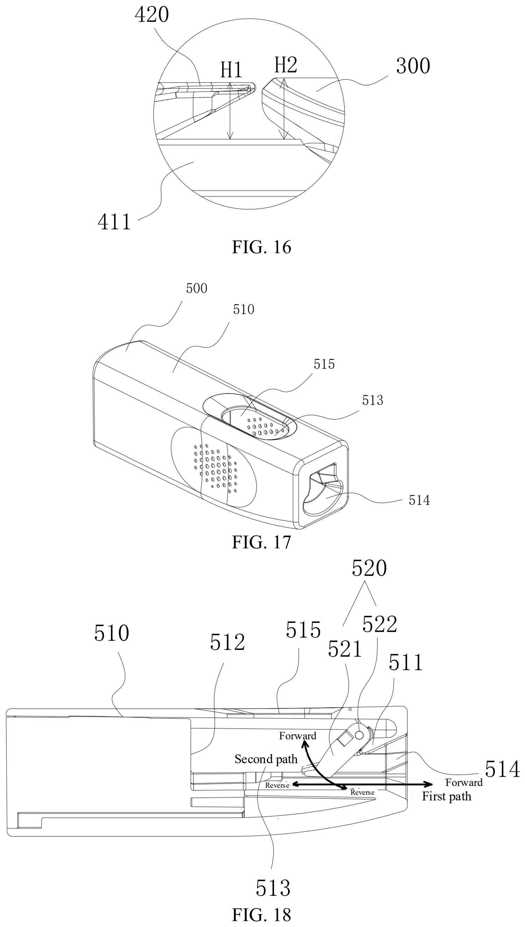

Please refer to FIG. 17 to FIG. 28, the disassembling tool 500 of this embodiment mainly includes a frame body 510 and a movement mechanism 520, wherein the movement mechanism 520 is in movable connection with the frame body 510.

The frame body 510 is configured to implement a forward movement and a reverse movement along a first path relative to the guide assembly 400 of the surgical instrument and the effector assembly 300 of the surgical instrument, during disassembling. A forward movement of the frame body 510 along the first path is capable of triggering the movement mechanism 520 to move along a second path relative to the guide assembly 400, and the reverse movement of the frame body 510 along the first path is capable of triggering the movement mechanism 520 to drive the guide assembly 400 and the effector assembly 300 of the surgical instrument to switch from a joined state to a separate state.

The first path is parallel to a joining direction of the guide assembly 400, and the first path is along a direction in which a combination of the guide assembly 400 and the effector assembly 300 moves relative to the frame body 510. The first path is a straight path, and the combination of the guide assembly 400 and the effector assembly 300 can implement a forward movement and a reverse movement along a straight line relative to the frame body 510, so as to disassemble the guide assembly 400.

The second path may be a circular path, and during disassembling, the movement mechanism 520 may only move along a partial arc segment of the circular path. The first path and the second path are located on a same plane and intersect with each other, so as to enable the movement mechanism 520 to move along the second path to disassemble the guide assembly 400, when the frame body 510 moves along the first path.

The disassembling tool only needs to implement a forward movement and a reverse movement along the first path, so as to realize disassembling of the guide assembly, that is, the disassembling tool can realize disassembling of the guide assembly through a back-and-forth pull-push action, and has advantages of high disassembling efficiency and of simple and convenient operation.

In an embodiment, the disassembling tool 500 includes two steps during disassembling: in the first step, the frame body 510 implements a forward movement along the first path, that is, the frame body 510 is relatively adjacent to the effector assembly 300; in the second step, the frame body 510 implement a reverse movement along the first path, that is, the frame body 510 is relatively far away from the effector assembly 300.

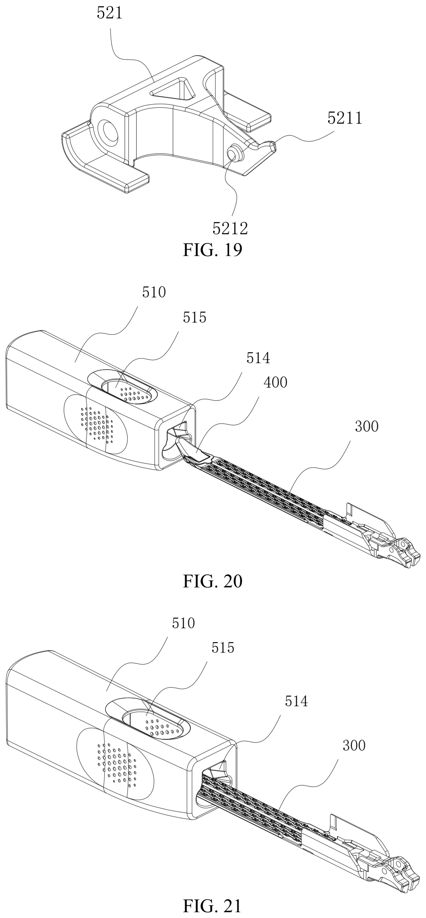

Please refer to FIGS. 20 to 24. In the first step: when the frame body 510 implements a forward movement along the first path, the guide assembly 400 passes over the movement mechanism 520 and drives the movement mechanism 520 to implement a forward movement along the second path. After the guide assembly 400 is in position, the guide assembly is located on an inner side of the movement mechanism 520, and the movement mechanism 520 abuts against the effector assembly 300. That is, the guide assembly 400 will move from an outer side to an inner side of the movement mechanism 520, and when being in contact with the movement mechanism 520, the guide assembly 400 will also drive the movement mechanism 520 to move. The outer side refers to that the guide assembly 400 and the effector assembly 300 are located at an outer side of the disassembling tool 500, and the inner side refers to that the guide assembly 400 is inserted into the disassembling tool 500.

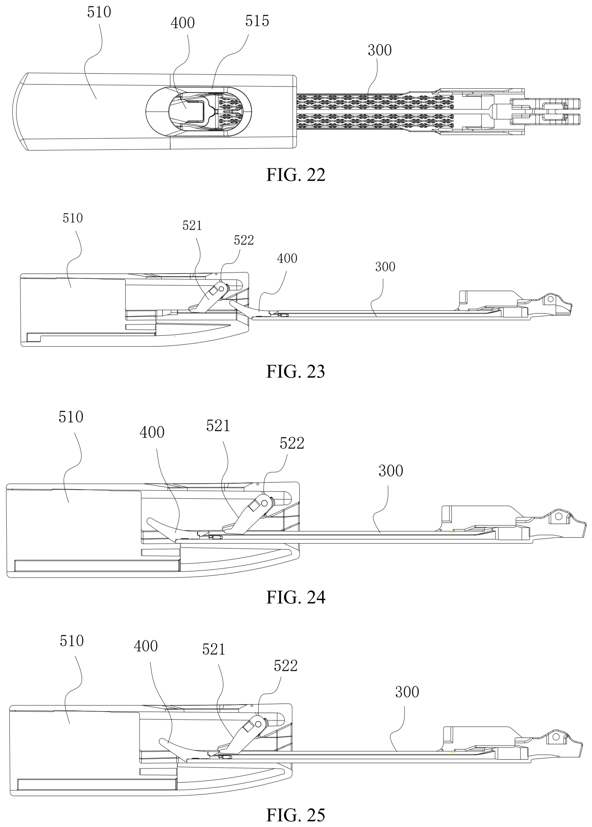

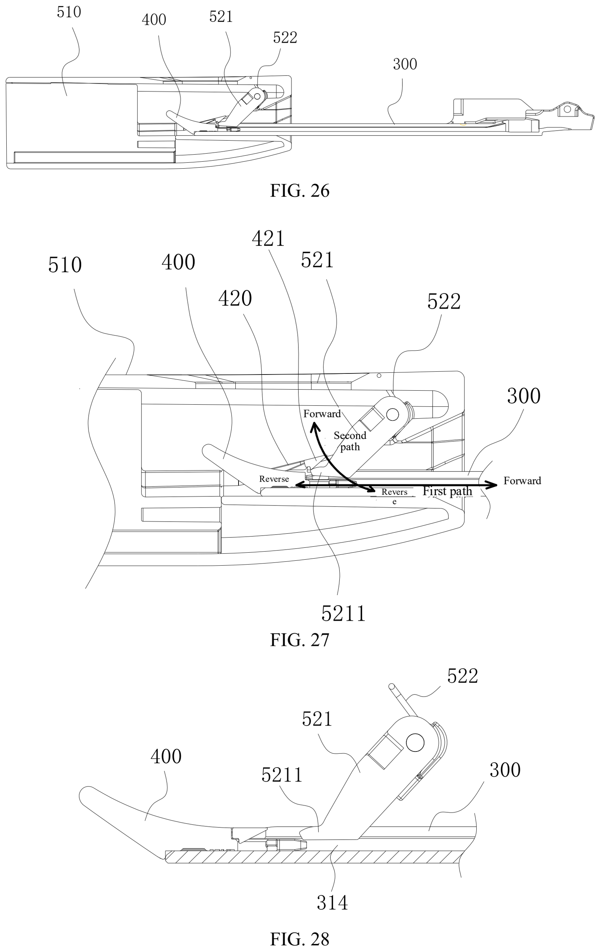

Please refer to FIGS. 25 and 27. In the second step: when the frame body 510 implements a reverse movement along the first path, the movement mechanism 520 abuts against the movable position-limiting member 420 of the guide assembly 400 and drives the movable position-limiting member 420 to move, so as to switch the movable position-limiting member 420 and the effector assembly 300 from the joined state to the separate state.

Wherein, the movement mechanism 520 can drive the movable position-limiting member 420 to move in a direction opposite to a snap-fit direction, so as to sperate the slidable snap-fit portion 421 of the movable position-limiting member 420 from the snap-fit structure 312 at the end of the effector assembly 300, and finally disassemble the guide assembly 400.

In an embodiment, the movement mechanism 520 may include a separating member 521 and a powering member 522, wherein the powering member 522 is connected with the separating member 521, the separating member 521 is configured to separate the slidable snap-fit portion 421 from the snap-fit structure 312, and the powering member 522 is configured to provide a resetting force to the separating member 521.

When the disassembling tool 500 disassembles, the frame body 510 implements a reverse movement (to the right in FIG. 27) along the first path, which drives the separating member 521 to implement a forward movement (clockwise in FIG. 27) along the second path. The powering member 522 is configured to apply a force on the separating member 521, so as to drive the separating member 521 to implement a reverse movement along the second path, so as to enable the separating member to abut against the knife slot 314 of the effector assembly 300. The frame body 510 implements a reverse movement (to the left in FIG. 27) along the first path, so as to trigger the separating member 521 to abut against the movable position-limiting member 420 and drive the movable position-limiting member 420 to move, so as to switch the movable position-limiting member 420 and the effector assembly 300 from the joined state to the separate state.

The separating member 521 may include a rod-shaped member, such as a switching lever, wherein the separating member 521 includes a connection end and an effector end, which are opposite to each other. The connection end of the separating member 521 may be in rotatable connection with the frame body 510, and the separating member 521 may be in rotatable connection with the frame body 510 via a rotation-shaft member. The effector end of the separating member 521 can implement a forward movement and a reverse movement along the second path. In the first step of a disassembling process, the guide assembly 400 will move from an outer side to an inner side of the effector end of the separating member 521, and drive the effector end of the separating member 521 to move to be snap-fitted into the knife slot 314 of the effector assembly 300; in the second step of the disassembling process, the effector end of the separating member 521 is arranged on a movement path of the slidable snap-fit portion 421 of the movable position-limiting member 420, so as to enable the effector end of the separating member 521 to contact with the slidable snap-fit portion 421, and drive the movable position-limiting member 420 to open relative to the main-body member 410, and finally switch the guide assembly 400 and the effector assembly 300 from the joined state to the separate state, thereby disassembling guide assembly 400.

Wherein, the powering member 522 can be an elastic member, such as a torsion spring, which can be installed at a rotation-shaft member, which connects the separating member 521 and the frame body 510. The powering member 522 always applies a torque to the separating member 521, such as a counterclockwise torque in FIG. 27, so as to drive the separating member 521 to implement a reverse movement along the second path. In this way, the separating member 521 can be squeezed and rotated by the guide assembly 400, and the guide assembly 400 passes over the separating member 521, and allows the effector end of the separating member 521 to abut against the knife slot 314 of the effector assembly 300, thereby finally disassembling the guide assembly 400.

In this embodiment, the separating member 521 is arranged as a rotating structure, and the separating member 521 is driven to reset by an elastic member, such as a torsion spring. The structure is relatively simple, which can reduce a production cost of the disassembling tool. In addition, a movement range of the separating member 521 is small, which has a high reliability and can ensure that the disassembling tool is not easily damaged for a long time.

In an embodiment, the effector end of the separating member 521 has a hook-shaped structure 5211, and the hook-shaped structure 5211 is located on a side, which side faces a forward direction of the second path, such as a left side in FIG. 28. The hook-shaped structure 5211 is configured to abut against the slidable snap-fit portion 421 to drive the movable position-limiting member 420 to move. In particular, when the abutted slidable snap-fit portion 421 is provided with a first guide surface, the hook-shaped structure 5211 at the effector end of the separating member 521 can guide the slidable snap-fit portion 421 to slide along the guide direction of the first guide surface, so as to drive the slidable snap-fit portion 421 to slide until it is separated from the snap-fit structure 312 of the effector assembly 300.

The effector end of the separating member 521 is provided with a hook-shaped structure 5211, which is capable of improving a stability of the separating member 521 in driving the movable position-limiting member 420 to move, and avoiding problems, such as the effector end of the separating member 521 and the movable position-limiting member 420 being stuck or slipped and misaligned.

In other embodiments, the effector end of the separating member 521 may also be another protruded structure, which is capable of abutting against the slidable snap-fit portion 421, so as to drive the slidable snap-fit portion 421 to slide to be separated from the snap-fit structure 312 of the effector assembly 300.

Please refer to FIGS. 18 and 28. In an embodiment, the frame body 510 is provided with a third position-limiting portion 511. The third position-limiting portion 511 can be a protruded structure. The third position-limiting portion 511 is arranged on a path, along which path the separating member 521 implement the reverse movement along the second path. The third position-limiting portion 511 is configured to limit the separating member 521 to an extreme position of a reverse movement along the second path. When the first step of the disassembling process is completed, and the separating member 521 is inserted into the knife slot 314 of the effector assembly 300, a gap exists between the effector end of the separating member 521 and a bottom surface of the knife slot 314, that is, the effector end of the separating member 521 does not contact with the bottom surface of the knife slot 314.

The gap between the effector end of the separating member 521 and the bottom surface of the knife slot 314 is arranged, so as to enable the separating member 521 not to come into contact with residual tissue and liquid on the bottom surface of the knife slot 314, thereby avoiding contamination of the disassembling tool by a used surgical instrument during disassembling, and avoiding residual tissue and liquid on the bottom surface of the knife slot 314 from affecting disassembling the guide assembly 400 of the separating member 521.

Please refer to FIGS. 19 and 28. In an embodiment, the effector end of the separating member 521 is provided with a fourth position-limiting portion 5212. The fourth position-limiting portion 5212 is a structure, which protrudes along a direction which direction is perpendicular to a plane, wherein the first path and the second path are located on said plane. The fourth position-limiting portion 5212 is configured to abut against the effector assembly 300, specifically for abutting against a top wall above the knife slot 314. The knife slot 314 is a T-shaped groove, and a top of the knife slot 314 is provided with a narrowed top wall. The fourth position-limiting portion 5212 abuts against an outer surface of the top wall above the knife slot 314, which is capable of implementing a position-limitation for the effector end of the separating member 521 relative to the knife slot 314.

A distance between the fourth position-limiting portion 5212 and the end of the effector end of the separating member 521 is smaller than a depth of the knife slot 314, so as to form a gap between the effector end of the separating member 521 and a bottom surface of the knife slot 314, when the effector end of the separating member 521 is inserted into the knife slot 314. In this way, the separating member 521 will not come into contact with residual tissue and liquid on the bottom surface of the knife slot 314, thereby avoiding contamination of the disassembling tool by a used surgical instrument during disassembling, and avoiding residual tissue and liquid on the bottom surface of the knife slot 314 from affecting disassembling the guide assembly 400 of the separating member 521.

The disassembling tool may be provided with a third position-limiting portion 511 and a fourth position-limiting portion 5212 at the same time, so as to forming a double position-limitation guarantee, which avoids a failure of a single position-limiting portion, which failure causes the effector end of the separating member 521 to contact with the bottom surface of the knife slot 314.

In other embodiments, only the third position-limiting portion 511 or the fourth position-limiting portion 5212 may be provided at the disassembling tool, which may also play a position-limiting role.

Please refer to FIG. 18, in an embodiment, the frame body 510 is provided with a fifth position-limiting portion 512, which is arranged on a path in which the guide assembly 400 moves along the first path.

The fifth position-limiting portion 512 can be a position-limiting structure, such as a position-limiting protrusion or a position-limiting plate. The fifth position-limiting portion 512 is configured to limit an extreme position for a reverse movement of the guide assembly 400 along the first path, so as to enable the guide assembly 400 to pass over the separating member 521 as a whole to position the guide assembly 400 on an inner side of the movement mechanism 520, when the first step of disassembling is completed, and so as to enable the movement mechanism 520 to abut against the movable limiting piece 420 of the guide assembly 400, when the second step of disassembling is carried out.

Please refer to FIG. 17 and FIG. 18. In an embodiment, the frame body 510 is an elongated rectangular structure. The frame body 510 may be an integrated structure, or may be formed by enclosing and fixing a plurality of plate structures.

The frame body 510 includes an accommodation cavity 513, wherein the movement mechanism 520 is movably assembled inside the accommodation cavity 513. The frame body 510 is provided with a first opening 514, which is located on an end surface of the frame body 510 along the length direction. The first opening 514 is connected with the accommodation cavity 513, and the first opening 514 is configured to enable the guide assembly 400 to enter into and exit from the accommodation cavity 513, so as to enable the guide assembly 400 to move inside the accommodation cavity 513 along the first path.

The movement mechanism 520 may be arranged at a position which is adjacent to the first opening 514, so as to enable the guide assembly 400 to move a shorter distance to pass over the movement mechanism 520, so as to achieve disassembling, thereby shortening the length of the frame body 510.

Please refer to FIGS. 17 and 18. In an embodiment, the frame body 510 may also be provided with a second opening 515, wherein the second opening 515 is arranged on one side of the frame body 510 along the length direction. The second opening 515 is configured to disassemble the guide assembly 400 from the accommodation cavity 513 of the frame body 510, after the guide assembly 400 is separated from the effector assembly 300. Furthermore, the second opening 515 can also be used as an observation window for the operator to check a separate state of the guide assembly 400, and to perform operations through the second opening 515, when a jam or another problem occurs.

Please refer to FIGS. 1 to 14, in an embodiment, a surgical instrument is provided, the surgical instrument includes a handle assembly 100, a barrel assembly 200, an effector assembly 300 and a guide assembly 400. The guide assembly 400 includes the guide assembly 400 in any one of the above-mentioned embodiments.

A proximal end of the barrel assembly 200 is connected with the handle assembly 100, and an end of the barrel assembly 200 is connected with the effector assembly 300.

The effector assembly 300 includes a first effector assembly 310 and a second effector assembly 320. The first effector assembly 310 is a staple anvil, and the second effector assembly 320 is a staple cartridge base. In this embodiment, the guide assembly 400 is joined with the first effector assembly 310, and the guide assembly 400 is arranged at the end of the first effector assembly 310. Of course, the guide assembly 400 can also be joined with the second effector assembly 320, and the guide assembly 400 can be arranged at the end of the second effector assembly 320; and both arrangements can guide the effector assembly 300 into a narrow space.

The first effector assembly 310 can be provided with a plug-in slot 311, a snap-fit structure 312 and a slidable guide portion 313. The first effector assembly 310 also includes a knife slot 314. The plug-in slot 311 is located at an end of the first effector assembly 310. The plug-in slot 311, the snap-fit structure 312 and the knife slot 314 are connected in sequence along the joining direction. The slidable guide portion 313 extends from the plug-in slot 311 to the snap-fit structure 312.

The sliding guide portion 313 can be a third guide surface. In a joined and aligned state of the guide assembly 400 and the effector assembly 300, the third guide surface is parallel to the first guide surface 4211, and the third guide surface and the first guide surface 4211 are parallel to the guide direction. The third guide surface can guide the first guide surface 4211 to slide along a planar direction of the third guide surface.

In other embodiments, one of the first guide surface 4211 and the third guide surface is a guide surface, and the other one is replaced by a guide protrusion. The guide protrusion can be a hemispherical or another arc structure. Fitting between the guide protrusion and the guide surface can also guide the movable position-limiting member 420 to slide along the joining direction, thereby realizing the slidable and elastic snap-fit of the movable position-limiting member 420.

When it is necessary to operate on tissue in a relatively narrow space, the operator can join the joining portion 411 of the guide assembly 400 with the end of the effector assembly 300 of the surgical instrument, so as to guide the effector assembly 300 into the tissue in the narrow space through the guide portion 412 of the guide assembly 400. When the guide assembly 400 is not needed, the operator can use a disassembling tool to move the movable position-limiting member 420 to move, so as to switch the guide assembly 400 and the surgical instrument from the joined state to the separate state, and then disassemble the guide assembly 400.

The operator is capable of satisfying a surgical requirement with only one surgical instrument with a guide assembly 400, without having to operate another instrument, which is conducive to reducing medical costs and expenses. Furthermore, since the slidable snap-fit portion 421 slides into and be snap-fitted with the snap-fit structure 312 of the effector assembly 300 through deformation, when the end of the effector assembly 300 operates, it is necessary to apply a deformation force to the slidable snap-fit portion 421, so as to switch from the joined state to the separate state, which is beneficial to prevent the guide assembly 400 from falling from the end of the effector assembly 300 during operation and reduce a risk of using the surgical instrument.

Please refer to FIG. 9 and FIG. 10. In an embodiment, the snap-fit structure 312 may be a circular hole, which is perpendicular to the joining direction. The snap-fit structure 312 may be a hole of other shapes, which hole is perpendicular to the joining direction, such as a square hole or an elliptical hole. The snap-fit structure 312 is perpendicular to the joining direction, so that after the slidable snap-fit portion 421 is snap-fitted with the snap-fit structure 312, the slidable snap-fit portion 421 cannot be automatically separated from the snap-fit structure 312, which can improve a stability of the snap-fit connection between the slidable snap-fit portion 421 and the snap-fit structure 312 and avoid separation of the guide assembly 400 and the effector assembly 300, during surgery.

Please refer to FIGS. 15 and 16. In an embodiment, a distance H1 between a surface of one end of the movable position-limiting member 420 and a second assembling surface of the joining portion 411 is smaller than a maximum distance H2 between a surface of the first effector assembly 310 (staple anvil) and a bottom surface of the plug-in slot 311 of the first effector assembly 310, that is, H1 < H2; wherein the one end of the movable position-limiting member 420 is provided with the slidable snap-fit portion 421; the surface of the one end of the movable position-limiting member 420 back-faces the joining portion 411; and the surface of the first effector assembly 310 (staple anvil) back-faces the second effector assembly 320 (staple cartridge base). Wherein, the joining portion 411 is provided with a first assembling surface and a second assembling surface, which are opposite to each other. The first assembling surface and the second assembling surface are parallel to each other, and both parallel to the joining direction. Under normal installation conditions, the first assembling surface contacts with the bottom surface of the plug-in slot 311.

After the guide assembly 400 rotates 180° along the joining direction, the second assembling surface of the joining portion 411 contacts with the bottom surface of the plug-in slot 311. At this time, since H1<H2, an end surface of the first effector assembly 310 abuts against the movable position-limiting member 420, so as to squeeze and move the movable position-limiting member 420, that is, the movable position-limiting member 420 will not be driven to move to form an open state, when it is plugged in along the joining direction, thereby preventing the guide assembly 400 from being reversely assembled at the first effector assembly 310. In other words, such a setting can play a role of reverse fool-proof assembling and avoid assembling errors by the operator.

In an embodiment, a surgical instrument is provided. The surgical instrument includes a handle assembly 100, a barrel assembly 200, an effector assembly 300, a guide assembly 400, and a disassembling tool 500. The guide assembly 400 includes the guide assembly 400 in any one of the above embodiments, and the disassembling tool 500 includes the disassembling tool 500 in any one of the above embodiments.

In an embodiment, a surgical instrument is provided, which includes a handle assembly 100, a barrel assembly 200, an effector assembly 300, a guide assembly 400, a disassembling tool 500 and a packaging assembly 600, wherein the guide assembly 400 includes the guide assembly 400 in any one of the above-mentioned embodiments, and the disassembling tool 500 includes the disassembling tool 500 in any one of the above-mentioned embodiments.

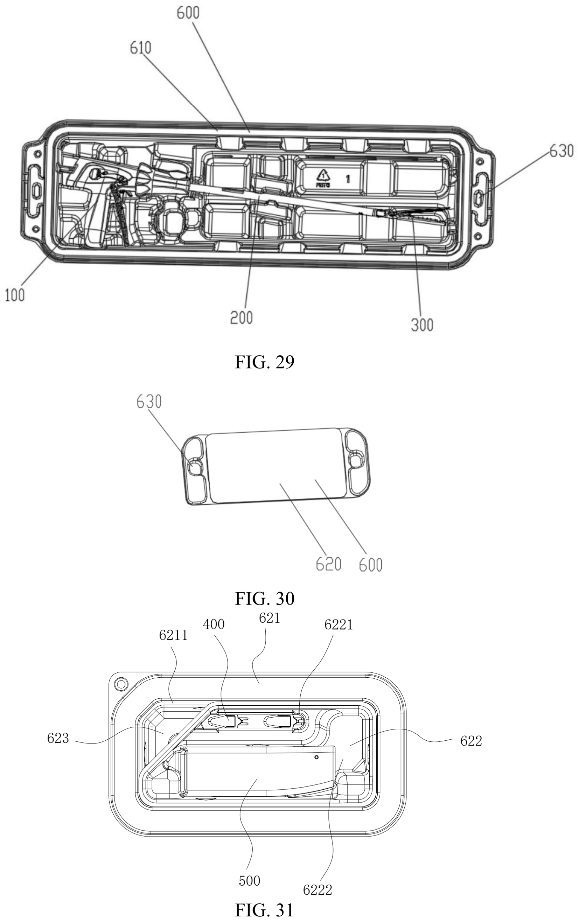

Please refer to FIGS. 29 and 30, the packaging assembly 600 includes a first packaging member 610 and a second packaging member 620, wherein the first packaging member 610 is configured to package the handle assembly 100, the barrel assembly 200 and the effector assembly 300, and the second packaging member 620 is configured to package the guide assembly 400 and the disassembling tool 500.

In an embodiment, the second packaging member 620 is connected with an upper surface, a lower surface, a side portion or an inner portion of the first packaging assembly 610. The first packaging member 610 and the second packaging member 620 may be connected together by bonding or other methods. This facilitates the operator to take out the first packaging member 610 and the second packaging member 620 at the same time.

In an embodiment, the second packaging member 620 is connected with the first packaging assembly 610 via a snap-in structure 630 of interference fit.

It is convenient for the operator to take out the first packaging member 610 and the second packaging member 620 at the same time. When the operator only needs to use member(s) inside the first packaging member 610, the first packaging member 610 can be separated from the second packaging member 620 conveniently.

In an embodiment, a first snap-fit portion may be provided on a lower surface of the first packaging member 610, and a second snap-fit portion may be provided on a lower surface of the second packaging member 620. One of the first snap-fit portion and the second snap-fit portion is a protrusion, and the other one is a slot. The first packaging member 610 and the second packaging member 620 can be in snap-in connection with each other, through a snap-fit connection between the protrusion and the slot, making it convenient for the operator to take out the first packaging member 610 and the second packaging member 620 at the same time.

In an embodiment, the lower surface of the first packaging member 610 is provided with a groove, and the second packaging member 620 can be assembled inside the groove of the lower surface of the first packaging member 610. The second packaging member 620 can be hidden inside the groove of the first packaging member 610 in whole or in part, so that when the first packaging member 610 and the second packaging member 620 are connected, an overall volume can be reduced, and a space occupied by the first packaging member 610 and the second packaging member 620 is reduced, which facilitates storage and packaging of the first packaging member 610 and the second packaging member 620.

Please refer to FIG. 31 and FIG. 32. In an embodiment, the second packaging member 620 includes an outer housing 621 and an inner liner 622. The outer housing 621 is provided inside with an accommodation cavity (inner cavity). The inner liner 622 is detachably arranged inside the accommodation cavity of the outer housing 621. The inner liner 622 is configured to accommodate at least one of the guide assembly and the disassembling tool into the accommodation cavity of the outer housing 621. When the guide assembly 400 or the disassembling tool 500 is needed, the inner liner 622, and the guide assembly 400 and the disassembling tool 500 which are located inside the inner liner 622, can be taken out first, and then the guide assembly 400 or the disassembling tool 500 can be taken out from the inner liner 622.

The guide assembly 400 and the disassembling tool 500 may be arranged side by side or stacked inside the inner liner 622.

An inner liner 622 is provided inside the second packaging 620, so as to enable the operator to take out the guide assembly 400 and the disassembling tool 500 as a whole from the packaging member and place them in a same position, which is convenient for the operator to use the guide assembly 400 and the disassembling tool 500 in turn, and allows the operator to quickly and accurately find the position of the guide assembly 400 and the disassembling tool 500, thereby improving interaction efficiency and interaction experience.

The inner liner 622 can also have a sterile environment. After the guide assembly 400 and the disassembling tool 500 are taken out from the second packaging member 620, they are still arranged inside the inner liner 622 with a sterile environment. This can prevent the guide assembly 400 and the disassembling tool 500 from being taken out and directly placed on a table, such as an operating table, thereby preventing cross-contamination between the guide assembly 400 and the disassembling tool 500 and the sterile environment.

Please refer to FIG. 31 and FIG. 32, in an embodiment, the inner liner 622 is provided with a first assembling groove 6221 and a second assembling groove 6222, which are located on one plane. A shape and a structure of the first assembling groove 6221 are adapted to those of the guide assembly 400. The first assembling groove 6221 is configured to assemble the guide assembly 400. The first assembling groove 6221 may have one or more first assembling grooves. A number of the first assembling grooves 6221 may be set according to usage requirements, so as to assemble a corresponding number of guide assemblies 400. The shape and the structure of the second assembling groove 6222 are adapted to those of the disassembling tool 500, and the second assembling groove 6222 is configured to assemble the disassembling tool 500.

The inner liner 622 may be a plastic member or a structure of other material, with a certain elastic energy. A position-limiting snap-fit may be formed between the first assembling groove 6221 and the guide assembly 400, so that the guide assembly 400 is not likely to fall out from the first assembling groove 6221. A position-limiting snap-fit may also be formed between the second assembling groove 6222 and the disassembling tool 500, so that the disassembling tool 500 is not likely to fall out from the second assembling groove 6222, thereby improving a safety of the inner liner 622 for assembling the guide assembly 400 and the disassembling tool 500.

Please refer to FIGS. 31 and 32. In an embodiment, The inner liner 622 may be provided with only a first assembling groove 6221 or only a second assembling groove 6222. The inner liner 622 is configured to separately assemble one of the guide assembly 400 and the disassembling tool 500, and the other of the guide assembly 400 and the disassembling tool 500 can be directly assembled inside the accommodation cavity of the outer housing 621.

In an embodiment, two inner liners 622 may be provided inside the outer housing 621, one inner liner 622 is provided with a first assembling groove 6221 for assembling the guide assembly 400; the other inner liner 622 is provided with a second assembling groove 6222 for assembling the disassembling tool 500. It is also possible to take out multiple guide assemblies 400 together, which is convenient for taking out, using and storing the multiple guide assemblies 400. In addition, it is also possible to avoid placing the guide assemblies 400 and the disassembling tools 500 directly on a sterile table, thereby preventing cross contamination.

Please refer to FIGS. 31 and 32. In an embodiment, a structure for taking and placing 623 is located at a joint between the inner liner 622 and the outer housing 621. The structure for taking and placing 623 is configured to facilitate taking out the inner liner 622 from the outer housing 621 and placing the inner liner 622 into the outer housing 621.

An inner wall of the accommodation cavity of the outer housing 621 may be provided with a placing step 6211. The placing step 6211 may be an annular step which surrounds the accommodation cavity. The placing step 6211 may also include a plurality of protrusions. A support portion is provided at an edge of the inner liner 622, and accommodated on the placing step 6211, so as to support the inner liner 622 inside the accommodation cavity of the outer housing 621. The support portion of the inner liner 622 may be provided with a notch, which notch enables a further notch to be formed between the edge of the inner liner 622 and the inner wall of the accommodation cavity of the outer housing 621. Said further notch is the structure for taking and placing 623. The operator may insert his finger into said further notch to take out the inner liner 622, so as to take out the inner liner 622 from the outer housing 621 and place the inner liner 622 into the outer housing 621.

The structure for taking and placing 623 is a notch structure, which requires no additional structure, thereby reduces an occupied space in the accommodation cavity of the outer housing 621.

In other embodiments, the structure for taking and placing 623 may also be a protruded structure provided at the inner liner 622, such as a protruded handle structure, and the operator may also take out the inner liner 622 from the outer housing 621 and place the inner liner 622 into the outer housing 621through the protruded structure.

The embodiments provided in FIGS. 33-60 are described below, and the structural features corresponding to reference numbers are different from those in FIGS. 1-32.

Please refer to FIG. 33. The stapler generally includes a handle assembly 100, a barrel assembly 200 and an effector assembly 300. The barrel assembly 200 includes a closed transmission chain and a firing transmission chain. The handle assembly 100 includes a fixed handle 110 and at least one trigger 120.

Please refer to FIGS. 33-60. This embodiment provides a surgical instrument.

Please refer to FIGS. 33-47, the surgical instrument includes a handle assembly 100, a barrel assembly 200, an effector assembly 300 and a guide assembly 400.

A proximal end of the barrel assembly 200 is connected with the handle assembly 100, and a distal end of the barrel assembly 200 is connected with the effector assembly 300. The effector assembly 300 includes a first end of the effector assembly 311 and a second end of the effector assembly 312. The first end of the effector assembly 311 and the second end of the effector assembly 312 are in movable connection with each other, wherein the first end of the effector assembly 311 and the second end of the effector assembly 312 are capable of moving relative to each other to close and open. The guide assembly 400 is capable of being joined with the first end of the effector assembly 311 or the second end of the effector assembly 312.

When it is necessary to operate on tissue in a relatively narrow space, the operator can join the guide assembly 400 with the first end of the effector assembly 311 or the second end of the effector assembly 312 of the surgical instrument, so as to guide the effector assembly 300 into the tissue in the narrow space through the guide assembly 400. The operator is capable of satisfying a surgical requirement with only one surgical instrument with a guide assembly 400, without having to operate another instrument, which is conducive to reducing medical costs and expenses.

On the other hand, this embodiment also provides a guide assembly for a surgical instrument.

Please refer to FIGS. 33-38, the guide assembly 400 includes a main-body member 410 and a movable position-limiting member 420.

The main-body member 410 includes a joining portion 411 and a guide portion 412. The joining portion 411 is joined with an end 310 of the effector assembly of the surgical instrument, and the guide portion 412 is arranged at the joining portion 411. The movable position-limiting member 420 is in movable connection with the joining portion 411 or the guide portion 412, and the movable position-limiting member 420 is capable of moving relative to the main-body member 410, so as to switch the joining portion 411 and the end 310 of the effector assembly between a separate state and a joined state. When the movable position-limiting member 420 is subjected to a force along a first direction, the joining portion 411 and the end 310 of the effector assembly are switched from a joined state to a separate state. The first direction is different from a direction of a reaction force, which force is applied by a working object to the end 310 of the effector assembly during working.

When it is necessary to operate on tissue in a relatively narrow space, the operator can join the joining portion 411 of the guide assembly 400 with the end 310 of the effector assembly of the surgical instrument, so as to guide the effector assembly 300 into the tissue in the narrow space through the guide portion 412 of the guide assembly 400. When the guide assembly 400 is not needed, the operator can operate the movable position-limiting member 420 to move, so as to switch the guide assembly 400 and the surgical instrument from the joined state to the separate state, and then disassemble the guide assembly 400.

On the one hand, the operator is capable of satisfying a surgical requirement with only one surgical instrument with a guide assembly 400, without having to operate another instrument, which is conducive to reducing medical costs and expenses. On the other hand, since the direction of the reaction force, which force is applied by the working object to the end 310 of the effector assembly during working, is different from the first direction, when the effector assembly 300 is used to clamp tissue, the joining portion 411 and the end 310 of the effector assembly are not easy to switch from the joined state to the separate state under the reaction force of the tissue, which is beneficial to prevent the guide assembly 400 from falling from the end 310 of the effector assembly during operation and reduce a risk of using the surgical instrument.

It should be noted that, in this embodiment, the end 310 of the effector assembly includes a first end of the effector assembly 311 and a second end of the effector assembly 312, the first end of the effector assembly 311 is a staple anvil 313, and the second end of the effector assembly 312 is a staple cartridge base 314.

Please refer to FIGS. 33-42. In an embodiment, an angle between the first direction and the direction of the reaction force is b, and a value range of the angle b is: 90°≤b≤180°.

Specifically, please refer to FIG. 42. The first direction in this embodiment is a direction indicated by arrow A in FIG. 42. When the angle b between the first direction and the direction of the reaction force is in a value range of 90°≤b≤180°, while the effector assembly 300 clamps tissue, the reaction force of the tissue will not form a component force along the first direction, so that the reaction force of the tissue will not cause the guide assembly 400 and the effector assembly 300 to switch from the joined state to the separate state, thereby preventing the guide assembly 400 from falling from the end 310 of the effector assembly during operation, and reducing a risk of using the surgical instrument.

Please refer to FIGS. 33-42, in an embodiment, the first direction is opposite to the direction of the reaction force.

Since the first direction is opposite to the direction of the reaction force, when the effector assembly 300 is used to clamp tissue, the reaction force of the tissue will not only not cause the guide assembly 400 and the effector assembly 300 to switch from the joined state to the separate state, but will also make the guide assembly 400 and the effector assembly 300 join more tightly, thereby helping to ensure stability and safety of the surgical instrument during operation.

Please refer to FIGS. 33-48. In an embodiment, the movable position-limiting member 420 is provided with snap-fit portion(s) 421, and the main-body member 410 and/or the effector assembly 300 are/is provided with a snap-fit structure 316, which is snap-fitted with the snap-fit portion 421. The snap-fit portion(s) 421 is(are) snap-fitted with the snap-fit structure(s) 316 in the joined state.

While the operator assembles the guide assembly 400 to the effector assembly 300, the operator can operate the movable position-limiting member 420 to move, so as to enable the snap-fit portion(s) 421 of the movable position-limiting member 420 to snap-fit with the snap-fit structure(s) 316 at the main-body member 410 and/or the effector assembly 300, thereby switching the guide assembly 400 and the end 310 of the effector assembly to the joined state. Specifically, the snap-fit portion 421 may be a snap-fit column 4211, a snap-fit rod 4212, a snap-fit block or a snap-fit structure 316 of another suitable shape, and the snap-fit structure 316 may be a snap-fit groove 3163 that is snap-fitted with the snap-fit portion 421. It should be noted that "the main-body member 410 and/or the effector assembly 300 are/is provided with a snap-fit structure 316" means that only the main-body member 410 is provided with the snap-fit structure 316, only the effector assembly 300 is provided with the snap-fit structure 316, or both the main-body member 410 and the effector assembly 300 are provided with the snap-fit structure 316.

Please refer to FIGS. 33-48. In an embodiment, the snap-fit portion 421 is configured as a snap-fit column 4211. The snap-fit structure 316 includes a first butt joint hole 3161, which perforates through the joining portion 411, and a second butt joint hole 3162, which is arranged at the end 310 of the effector assembly. In the joined state, the snap-fit column 4211 penetrates through the first butt joint hole 3161 and the second butt joint hole 3162, so as to join and lock the joining portion 411 with the end 310 of the effector assembly.

While the operator assembles the guide assembly 400 to the effector assembly 300, the operator can operate the movable position-limiting member 420 to move, so as to enable the snap-fit column 4211 at the movable position-limiting member 420 to penetrate through the first butt joint hole 3161 and the second butt joint hole 3162, so as to join the guide assembly 400 and the end 310 of the effector assembly. Specifically, a number and a position of the snap-fit portion 421 can be arranged arbitrarily according to actual conditions. For example, please refer to FIG. 35. In FIG. 35, the number of the snap-fit portion 421 is one, and said snap-fit portion 421 is located in a middle of the movable position-limiting member 420. For another example, please refer to FIG. 54, in which, there are two snap-fit portions 421, which are respectively located on two sides of the movable position-limiting member 420.

Please refer to FIG. 56. In an embodiment, the snap-fit portion 421 is configured as a snap-fit rod 4212. The snap-fit rod 4212 is snap-fitted with the snap-fit groove 3163 at the end 310 of the effector assembly in the joined state.

While the operator assembles the guide assembly 400 to the effector assembly 300, the operator can operate the movable position-limiting member 420 to move, so as to enable the snap-fit rod 4212 on the movable position-limiting member 420 to snap-fit with the snap-fit groove 3163 on the end 310 of the effector assembly, so as to join the guide assembly 400 and the end 310 of the effector assembly. Of course, in other embodiments, the snap-fit portion 421 can also be configured as a block structure, a strip structure, a circular structure, a triangular structure or other suitable shapes. Please refer to FIG. 57. The snap-fit portion 421 can even be configured as an irregular structure.

Please refer to FIGS. 33-48. In an embodiment, the joining portion 411 is a plug-in structure 4111, and the plug-in structure 4111 is plugged into and fitted with the plug-in slot 317 on the end 310 of the effector assembly.

While the operator assembles the guide assembly 400 to the effector assembly 300, the operator can first plug the plug-in structure 4111 of the joining portion 411 into the plug-in slot 317 at the end 310 of the effector assembly, and then operate the movable position-limiting member 420 to move, so as to guide the guide assembly 400 and the end 310 of the effector assembly to switch to a joined state. Specifically, the plug-in structure 4111 may be a sheet-like structure, a strip-like structure or other suitable shapes.

Please refer to FIGS. 33-48. In an embodiment, the movable position-limiting member 420 is capable of rotating relative to the main-body member 410.

While the operator assembles the guide assembly 400 to the effector assembly 300, the operator can operate the movable position-limiting member 420 to rotate, so as to enable the snap-fit portion(s) 421 of the movable position-limiting member 420 to snap-fit with the snap-fit structure(s) 316 at the main-body member 410 and/or the effector assembly 300, so as to switch the guide assembly 400 and the end 310 of the effector assembly to a joined state.

Specifically, please refer to FIGS. 33-48. When the operator rotates the movable position-limiting member 420 along a direction R1 in FIG. 34, the snap-fit portion 421 of the movable position-limiting member 420 can be snap-fitted with the snap-fit structure 316 at the main-body member 410 and/or the effector assembly 300. When the operator rotates the movable position-limiting member 420 along a direction R2 in FIG. 34, the snap-fit portion 421 of the movable position-limiting member 420 can be released from the snap-fit structure 316 at the main-body member 410 and/or the effector assembly 300. Of course, in other embodiments, the connection method between the movable position-limiting member 420 and the main-body member 410 may also be a sliding connection or another suitable connection method.

Please refer to FIGS. 33-48. In an embodiment, the main-body member 410 is provided with a rotation-shaft hole 413, and the movable position-limiting member 420 is provided with a rotation-shaft portion 424, which fits with the rotation-shaft hole 413, so as to enable the movable position-limiting member 420 to rotate relative to the main-body member 410 through the rotation-shaft portion 424. The rotation connection between the movable position-limiting member 420 and the main-body member 410 is achieved through the fitting between the rotation-shaft portion 424 and the rotation-shaft hole 413. In other embodiments, the rotatable connection between the movable position-limiting member 420 and the main-body member 410 may also be achieved through a hinge, a bearing or other suitable structures.

Please refer to FIGS. 33-48. In an embodiment, a side portion of the main-body member 410 is provided with a first position-limiting portion 414 and a second position-limiting portion 415, which are protruded thereat. The first position-limiting portion 414 and the second position-limiting portion 415 are configured to fit with the movable position-limiting portion 420 in the joined state, so as to prevent the movable position-limiting portion 420 from moving relative to the main-body member 410. The first position-limiting portion 414 and the second position-limiting portion 415 limit a rotation angle of the movable position-limiting member 420, so as to prevent the movable position-limiting member 420 from rotating to an angle, that is inconvenient for the operator to operate.

Please refer to FIGS. 33-48. In an embodiment, two opposite side portions of the main-body member 410 are respectively provided with a rotation-shaft hole 413, and the movable position-limiting member 420 is provided with two rotation-shaft portions 424. The rotation-shaft portions 424 are fitted with the rotation-shaft holes 413 in a one-to-one correspondence, so as to enable the movable position-limiting member 420 to rotate relative to the main-body member 410 through the rotation-shaft portions 424. Two opposite sides of the main-body member 410 are respectively provided with a first position-limiting portion 414 and a second position-limiting portion 415, which are protruded thereat and configured to fit with the movable position-limiting portion 420 in the joined state, so as to prevent the movable position-limiting portion 420 from moving relative to the main-body member 410.

On the one hand, through the fitting between the two rotation-shaft portions 424 and the two rotation-shaft holes 413, the rotatable connection between the movable position-limiting member 420 and the main-body member 410 is made more stable and reliable. On the other hand, a first position-limiting portion 414 and a second position-limiting portion 415 are respectively arranged on two opposite sides of the main-body member 410, which can more effectively limit the rotation angle of the movable position-limiting member 420 and prevent the movable position-limiting member 420 from rotating to an angle, which is inconvenient for the operator to operate.

In an embodiment, the movable position-limiting member 420 is provided with an elastic fitting portion (not shown) on a side, which side faces the main-body member 410, and the elastic fitting portion can be elastically deformed under an external force, so as to switch the movable position-limiting member 420 and the main-body member 410 between a fixed state and a movable state.

The movable position-limiting member 420 and the main-body member 410 can be switched between a fixed state and a movable state by a deformation of the elastic fitting portion. For example, the movable position-limiting member 420 and the main-body member 410 can be fixed by expansion of the elastic fitting portion, and can be movable relative to each other by a contraction of the elastic fitting portion.

Refer to FIGS. 33-48, in an embodiment, the main-body member 410 is joined with the end 310 of the effector assembly, so as to form a smooth contact surface for tissue. This helps to reduce friction between the main-body member 410 and the end 310 of the effector assembly and the tissue, after the main-body member 410 and the end 310 of the effector assembly are joined, thereby helping to improve experience and surgical efficiency of the operator.

On the other hand, this embodiment also provides a guide assembly for a surgical instrument.

Please refer to FIGS. 33-48 and 49-52. The guide assembly 400 includes a main-body member 410 and a movable position-limiting member 420. The main-body member 410 includes a joining portion 411 and a guide portion 412. The joining portion 411 is joined with the end 310 of the effector assembly of the surgical instrument. The guide portion 412 is arranged at the joining portion 411. The movable position-limiting member 420 is in movable connection with the main-body member 410, and the movable position-limiting member 420 is capable of moving relative to the main-body member 410, so as to switch the joining portion 411 and the end 310 of the effector assembly between a separate state and a joined state. The movable position-limiting member 420 is provided with an assembling portion 422 and a disassembling portion 423, wherein the disassembling portion 423 and the assembling portion 422 are located at different positions of the movable position-limiting member 420. In the joined state, the movable position-limiting member 420 is configured to join and lock the joining portion 411 with the end 310 of the effector assembly. The disassembling portion 423 fits with the disassembling tool 500, so as to switch the joining portion 411 and the end 310 of the effector assembly from a joined state to a separate state.

When it is necessary to operate on tissue in a relatively narrow space, the operator can join the joining portion 411 of the guide assembly 400 with the end 310 of the effector assembly of the surgical instrument, so as to guide the effector assembly 300 into the tissue in the narrow space through the guide portion 412 of the guide assembly 400. When the guide assembly 400 is not needed, the operator can operate the movable position-limiting member 420 to move, so as to switch the guide assembly and the surgical instrument from a joined state to a separate state, and then disassemble the guide assembly. On the one hand, the operator is capable of satisfying a surgical requirement with only one surgical instrument with a guide assembly, without having to operate another instrument, which is conducive to reducing medical costs and expenses. On the other hand, since the disassembling portion 423 and the assembling portion 422 are located at different positions of the movable position-limiting member 420, a division of different functional areas of the movable position-limiting member 420 is clearer, which facilitates the operator to perform accurate operations and helps to reduce a probability of disoperation. The operator can use the disassembling tool 500 to fit with the disassembling portion 423 of the movable position-limiting member 420, so as to switch the joining portion 411 and the end 310 of the effector assembly from a joined state to a separate state.

Please refer to FIGS. 33-48 and 49-52. In an embodiment, the main-body member 410 and/or the effector assembly 300 are/is provided with a snap-fit structure 316 that is snap-fitted with the assembling portion 422, and the assembling portion 422 is snap-fitted with the snap-fit structure(s) 316 in the joined state.

While the operator assembles the guide assembly 400 to the effector assembly 300, the operator can operate the movable position-limiting member 420 to move, so as to enable the assembling portion 422 of the movable position-limiting member 420 to snap-fit with the snap-fit structure(s) 316 at the main-body member 410 and/or the effector assembly 300, thereby switching the guide assembly 400 and the end 310 of the effector assembly to the joined state. It should be noted that, referring to FIG. 35, the assembling portion 422 can be a same member as the above-mentioned snap-fit portion 421, or it can be an additional member which is independent of the snap-fit portion 421 and used to achieve connection between the movable position-limiting member 420 and the main-body member 410 and/or the effector assembly 300.

Please refer to FIGS. 33-48 and 49-52. In an embodiment, the assembling portion 422 is configured as a snap-fit column 4211. The snap-fit structure 316 includes a first butt joint hole 3161, which perforates through the joining portion 411, and a second butt joint hole 3162, which is arranged at the end 310 of the effector assembly. In the joined state, the snap-fit column 4211 penetrates through the first butt joint hole 3161 and extends into the second butt joint hole 3162, so as to join and lock the joining portion 411 with the end 310 of the effector assembly.

While the operator assembles the guide assembly 400 to the effector assembly 300, the operator can operate the movable position-limiting member 420 to move, so as to enable the snap-fit column 4211 at the movable position-limiting member 420 to penetrate through the first butt joint hole 3161 and extend into the second butt joint hole 3162, so as to join the guide assembly 400 and the end 310 of the effector assembly.

On the other hand, this embodiment also provides a surgical instrument.

Refer to FIGS. 33-48 and 49-52, the surgical instrument includes a handle assembly 100, a barrel assembly 200, an effector assembly 300, and a guide assembly 400.

A proximal end of the barrel assembly 200 is connected with the handle assembly 100, and a distal end of the barrel assembly 200 is connected with the effector assembly 300. The effector assembly 300 includes a staple anvil 313 and a staple cartridge base 314. The staple anvil 313 and the staple cartridge base 314 are in movable connection with each other. The staple anvil 313 and the staple cartridge base 314 are capable of moving relative to each other, so as to close and open. The guide assembly 400 includes a main-body member 410 and a movable position-limiting member 420. The main-body member 410 includes a joining portion 411 and a guide portion 412. The joining portion 411 is joined with the effector assembly tip 310 of a surgical instrument. The guide portion 412 is arranged at the joining portion 411. The movable position-limiting member 420 is in movable connection with the main-body member 410, and the movable position-limiting member 420 is capable of moving relative to the main-body member 410, so as to switch the joining portion 411 and the staple anvil 313 between a separate state and a joined state. The movable position-limiting member 420 is provided with an assembling portion 422 and a disassembling portion 423, and the disassembling portion 423 and the assembling portion 422 are located at different positions of the movable position-limiting member 420. In the joined state, the movable position-limiting member 420 is configured to join and lock the joining portion 411 with the end 310 of the effector assembly. The disassembling portion 423 fits with the disassembling tool 500, so as to switch the joining portion 411 and the end 310 of the effector assembly from a joined state to a separate state.

When it is necessary to operate on tissue in a relatively narrow space, the operator can join the joining portion 411 of the guide assembly 400 with the end 310 of the effector assembly of the surgical instrument, so as to guide the effector assembly 300 into the tissue in the narrow space through the guide portion 412 of the guide assembly 400. When the guide assembly 400 is not needed, the operator can operate the movable position-limiting member 420 to move, so as to switch the guide assembly and the surgical instrument from a joined state to a separate state, and then disassemble the guide assembly. On the one hand, the operator is capable of satisfying a surgical requirement with only one surgical instrument with a guide assembly, without having to operate another instrument, which is conducive to reducing medical costs and expenses. On the other hand, since the disassembling portion 423 and the assembling portion 422 are located at different positions of the movable position-limiting member 420, a division of different functional areas of the movable position-limiting member 420 is clearer, which facilitates the operator to perform accurate operations and helps to reduce a probability of disoperation. The operator can use the disassembling tool 500 to fit with the disassembling portion 423 of the movable position-limiting member 420, so as to switch the joining portion 411 and the end 310 of the effector assembly from a joined state to a separate state.

Please refer to FIGS. 49-52. In an embodiment, a side portion of the staple anvil 313 is provided with a recessed portion 315, which is recessed inward from an edge. In the joined state, the disassembling portion 423 of the movable position-limiting member extends to the recessed portion 315. The recessed portion 315 is configured to enable a movement mechanism 520 of the disassembling tool 500 to extend into and contact with the disassembling portion 423, and configured to drive the movable position-limiting member to move relative to the main-body member 410, so as to switch the joining portion 411 and the staple anvil 313 from the joined state to the separate state.

Since the disassembling portion 423 of the movable position-limiting member 420 extends to the recessed portion 315, it is convenient for the operator to extend the movement mechanism 520 of the disassembling tool 500 from the recessed portion 315 to contact with the disassembling portion 423, and drive the movable position-limiting member 420 member to move relative to the main-body member 410, so as to switch the joining portion 411 and the staple anvil 313 from the joined state to the separate state. Of course, in other embodiments, the edge of the staple anvil 313 may not be provided with the recessed portion 315, but the movable position-limiting member 420 may be extended, so as to enable the movable position-limiting member 420 to extend from the edge of the staple anvil 313 to match with the disassembling tool 500.

Please refer to FIG. 51 and 52, in an embodiment, two opposite side portions of the staple anvil 313 are respectively provided with a recessed portion 315. This facilitates the operator to use the disassembling tool 500 to more stably push the movable position-limiting member 420 from both sides of the staple anvil 313 to move, so as to switch the joining portion 411 and the staple anvil 313 from the joined state to the separate state.

Please refer to FIGS. 51 and 52. In an embodiment, the recessed portion 315 is configured in a stepped or arc shape. Specifically, please refer to FIG. 51, the recessed portion 315 in FIG. 51 is configured in a stepped shape, and please refer to FIG. 52, the recessed portion 315 in FIG. 52 is configured in an arc shape. Of course, in other embodiments, according to actual application scenario and structural strength requirements, the recessed portion 315 may also be configured as a trapezoid shape, a triangle shape or other suitable shapes.

Please refer to FIGS. 33-48 and 49-52. In an embodiment, the main-body member 410 and/or the effector assembly 300 are/is provided with a snap-fit structure 316 that is snap-fitted with the snap-fit portion 421, and the assembling portion 422 is snap-fitted with the snap-fit structure(s) 316 in the joined state.

While the operator assembles the guide assembly 400 to the effector assembly 300, the operator can operate the movable position-limiting member 420 to move, so as to enable the assembling portion 422 of the movable position-limiting member 420 to snap-fit with the snap-fit structure(s) 316 at the main-body member 410 and/or the effector assembly 300, thereby switching the guide assembly 400 and the end 310 of the effector assembly to the joined state. It should be noted that, referring to FIG. 34 and FIG. 35, the assembling portion 422 can be a same member as the above-mentioned snap-fit portion 421, or it can be an additional member which is independent of the snap-fit portion 421 and used to achieve connection between the movable position-limiting member 420 and the main-body member 410 and/or the effector assembly 300.

Please refer to FIGS. 33-48 and 49-52. In an embodiment, the assembling portion 422 is configured as a snap-fit column 4211. The snap-fit structure 316 includes a first butt joint hole 3161, which perforates through the joining portion 411, and a second butt joint hole 3162, which is arranged at the end 310 of the effector assembly. In the joined state, the snap-fit column 4211 penetrates through the first butt joint hole 3161 and extends into the second butt joint hole 3162, so as to join and lock the joining portion 411 with the end 310 of the effector assembly.

While the operator assembles the guide assembly 400 to the effector assembly 300, the operator can operate the movable position-limiting member 420 to move, so as to enable the snap-fit column 4211 at the movable position-limiting member 420 to penetrate through the first butt joint hole 3161 and extend into the second butt joint hole 3162, so as to join the guide assembly 400 and the end 310 of the effector assembly.

Please refer to FIGS. 48 and 51. In an embodiment, the second butt joint hole 3162 is a blind hole, wherein one end of the blind hole, which end is away from the movable position-limiting member 420, is closed.

Since the disassembling portion 423 and the assembling portion 422 are located at different positions, there is no need to disassemble the movable position-limiting member 420 from the second butt joint hole 3162, so that the second butt joint hole 3162 can be arranged as a blind hole, which is beneficial to improving a structural strength of the staple anvil 313. Of course, in other embodiments, the second butt joint hole 3162 may also be configured as a through hole.

On the other hand, this embodiment also provides a guide assembly for a surgical instrument.

Please refer to FIGS. 33-48, the guide assembly 400 includes a main-body member 410 and a movable position-limiting member 420. The main-body member 410 includes a joining portion 411 and a guide portion 412. The joining portion 411 is joined with the distal end 310 of the surgical instrument effector assembly. The guide portion 412 is arranged at the joining portion 411. The movable position-limiting member 420 is in movable connection with the main-body member 410, and the movable position-limiting member 420 is capable of moving relative to the main-body member 410, so as to switch the joining portion 411 and the end 310 of the effector assembly between a separate state and a joined state. In the joined state, the joining portion 411 is rigidly connected with the end 310 of the effector assembly, and the movable position-limiting member 420 is located on a surface, on which surface a staple anvil 313 of the effector assembly 300 joins with tissue.

When it is necessary to operate on tissue in a relatively narrow space, the operator can join the joining portion 411 of the guide assembly 400 with the end 310 of the effector assembly of the surgical instrument, so as to guide the effector assembly 300 into the tissue in the narrow space through the guide portion 412 of the guide assembly 400. When the guide assembly 400 is not needed, the operator can operate the movable position-limiting member 420 to move, so as to switch the guide assembly and the surgical instrument from a joined state to a separate state, and then disassemble the guide assembly. On the one hand, the operator is capable of satisfying a surgical requirement with only one surgical instrument with a guide assembly, without having to operate another instrument, which is conducive to reducing medical costs and expenses. On the other hand, since the joining portion 411 is rigidly connected with the end 310 of the effector assembly in the joined state, an overall structural strength of the guide assembly 400 and the effector assembly 300 is higher after being joined. Furthermore, the movable position-limiting member 420 is located on a surface, on which surface a staple anvil 313 of the effector assembly 300 joins with tissue, so that when the operator uses the effector assembly 300 to clamp the tissue, the joining portion 411 and the end 310 of the effector assembly are not easily switched from the joined state to the separate state under the reaction force of the tissue, thereby preventing the guide assembly 400 from falling from the end 310 of the effector assembly during operation and reducing a risk of using the surgical instrument.

Please refer to FIGS. 33-48. In an embodiment, the movable position-limiting member 420 is provided with snap-fit portion(s) 421, and the main-body member 410 and/or the effector assembly 300 are/is provided with a snap-fit structure 316, which is snap-fitted with the snap-fit portion 421. The snap-fit portion(s) 421 is(are) snap-fitted with the snap-fit structure(s) 316 in the joined state.

While the operator assembles the guide assembly 400 to the effector assembly 300, the operator can operate the movable position-limiting member 420 to move, so as to enable the snap-fit portion(s) 421 of the movable position-limiting member 420 to snap-fit with the snap-fit structure(s) 316 at the main-body member 410 and/or the effector assembly 300, thereby switching the guide assembly 400 and the end 310 of the effector assembly to the joined state.

Please refer to FIGS. 33-48. In an embodiment, the snap-fit portion 421 is configured as a snap-fit column 4211. The snap-fit structure 316 includes a first butt joint hole 3161, which perforates through the joining portion 411, and a second butt joint hole 3162, which is arranged at the end 310 of the effector assembly. In the joined state, the snap-fit column 4211 penetrates through the first butt joint hole 3161 and the second butt joint hole 3162, so as to join and lock the joining portion 411 with the end 310 of the effector assembly.

While the operator assembles the guide assembly 400 to the effector assembly 300, the operator can operate the movable position-limiting member 420 to move, so as to enable the snap-fit column 4211 at the movable position-limiting member 420 to penetrate through the first butt joint hole 3161 and the second butt joint hole 3162, so as to join the guide assembly 400 and the end 310 of the effector assembly.

On the other hand, the embodiment also provides a guide assembly for a surgical instrument.

Please refer to FIGS. 33-48, the guide assembly 400 includes a main-body member 410 and a movable position-limiting member 420. The main-body member 410 includes a joining portion 411 and a guide portion 412. The joining portion 411 is joined with the effector assembly tip 310 of a surgical instrument. The guide portion 412 is arranged at the joining portion 411. A cross-section area of the guide portion 412 gradually decreases from a proximal end of the guide portion 412 to a distal end of the guide portion 412. A central axis of the guide portion 412 along the length direction and a central axis of the effector assembly 300 along the length direction form an angle a. A value range of the angle a is: 5°≤a≤45°. The movable position-limiting member 420 is in movable connection with the joining portion 411 or the guide portion 412, and the movable position-limiting member 420 is capable of moving relative to the main-body member 410, so as to switch the joining portion 411 and the end 310 of the effector assembly between a separate state and a joined state. In the joined state, the movable position-limiting member 420 is joined with the end 310 of the effector assembly to form a smooth surface to contact with tissue.

When it is necessary to operate on tissue in a relatively narrow space, the operator can join the joining portion 411 of the guide assembly 400 with the end 310 of the effector assembly of the surgical instrument, so as to guide the effector assembly 300 into the tissue in the narrow space through the guide portion 412 of the guide assembly 400. When the guide assembly 400 is not needed, the operator can operate the movable position-limiting member 420 to move, so as to switch the guide assembly and the surgical instrument from a joined state to a separate state, and then disassemble the guide assembly. On the one hand, the operator is capable of satisfying a surgical requirement with only one surgical instrument with a guide assembly, without having to operate another instrument, which is conducive to reducing medical costs and expenses. On the one hand, the operator is capable of satisfying a surgical requirement with only one surgical instrument with a guide assembly 400, without having to operate another instrument, which is conducive to reducing medical costs and expenses. On the other hand, since the angle between the central axis of the guide portion 412 along the length direction and the central axis of the effector assembly 300 along the length direction is reasonably designed, the effector assembly 300 can be more effectively guided into a narrower tissue.

Please refer to FIGS. 33-48. In an embodiment, the movable position-limiting member 420 is provided with snap-fit portion(s) 421, and the main-body member 410 and/or the effector assembly 300 are/is provided with a snap-fit structure 316, which is snap-fitted with the snap-fit portion 421. The snap-fit portion(s) 421 is(are) snap-fitted with the snap-fit structure(s) 316 in the joined state.

While the operator assembles the guide assembly 400 to the effector assembly 300, the operator can operate the movable position-limiting member 420 to move, so as to enable the snap-fit portion(s) 421 of the movable position-limiting member 420 to snap-fit with the snap-fit structure(s) 316 at the main-body member 410 and/or the effector assembly 300, thereby switching the guide assembly 400 and the end 310 of the effector assembly to the joined state.

Please refer to FIGS. 33-48. In an embodiment, the snap-fit portion 421 is configured as a snap-fit column 4211. A movement plane of the snap-fit column 4211 is parallel to an axis of the main-body member 410. The joining portion 411 is provided with a first butt joint hole 3161 which is a through hole, and the first butt joint hole 3161 is aligned with the second butt joint hole 3162 at the end 310 of the effector assembly of the surgical instrument. In the joined state, the snap-fit column 4211 penetrates through the first butt joint hole 3161 and the second butt joint hole 3162, so as to join the joining portion 411 with the end 310 of the effector assembly.

While the operator assembles the guide assembly 400 to the effector assembly 300, the operator can operate the movable position-limiting member 420 to move, so as to enable the snap-fit column 4211 at the movable position-limiting member 420 to penetrate through the first butt joint hole 3161 and the second butt joint hole 3162, thereby joining the guide assembly 400 and the end 310 of the effector assembly.

On the other hand, the embodiment also provides a disassembling tool for a guide assembly for a surgical instrument.

Please refer to FIGS. 33-54, the disassembling tool 500 includes a frame body 510 and a movement mechanism 520.

The frame body 510 is configured to move along a first path relative to the guide assembly 400 and the effector assembly 300 of the surgical instrument, while the disassembling tool disassembles the guide assembly. The movement mechanism 520 is in movable connection with the frame body 510, and a movement of the frame body 510 along the first path is capable of triggering the movement mechanism 520 to move along a second path relative to the frame body 510, so as to drive the guide assembly 400 and the effector assembly 300 of the surgical instrument to switch from a joined state to a separate state.

When it is necessary to disassemble the guide assembly 400 at the effector assembly 300, the operator can align the disassembling tool 500 with the guide assembly 400 and operate the disassembling tool 500 to move along a first path to trigger the movement mechanism 520 to move along a second path relative to the frame body 510, and drive the guide assembly 400 and the effector assembly 300 of the surgical instrument to switch from a joined state to a separate state through the movement mechanism 520, thereby disassembling the guide assembly 400.

Please refer to FIGS. 39-44, in an embodiment, the first path is different from the second path.

By designing the first path and the second path differently, a possibility of mutual interference between the frame body 510 and the movement mechanism 520 during their movements can be reduced. Specifically, in this embodiment, the first path is a straight path shown by an axis L1 in FIG. 39, and the second path is a circular path shown by an arrow R3 in FIG. 42.

Please refer to FIGS. 39-44. In an embodiment, when the frame body 510 moves along the first path relative to the effector assembly 300, the frame body 510 drives the movement mechanism 520 to contact with the effector assembly 300 and/or the guide assembly 400, so as to trigger the movement mechanism 520. When the movement mechanism 520 is triggered, it moves along the second path and applies a continuous force to the movable position-limiting member 420 of the guide assembly 400, so as to switch the movable position-limiting member 420 and the effector assembly 300 from the joined state to the separate state.

When it is necessary to disassemble the guide assembly 400 at the effector assembly 300, the operator can align the disassembling tool 500 with the guide assembly 400 and operate the disassembling tool 500 to move along a first path to trigger the movement mechanism 520 to move along a second path relative to the frame body 510, and apply a continuous force to the movable position-limiting member 420 of the guide assembly 400 through the guide assembly 400, so as to switch the movable position-limiting member 420 and the effector assembly 300 from the joined state to the separate state.

Please refer to FIGS. 39-44. In an embodiment, the movement mechanism 520 is in rotatable connection with the frame body 510. The movement mechanism 520 includes a contact portion 521 and an effector portion 522. When the frame body 510 moves along a first path relative to the effector assembly 300, the contact portion 521 is driven to contact with the guide assembly 400, so as to enable the effector portion 522 to rotate along the second path relative to the frame body 510. The effector portion 522 is configured to apply a force on the movable position-limiting member 420 of the guide assembly 400, so as to switch the movable position-limiting member 420 and the effector assembly 300 from the joined state to the separate state.

When it is necessary to disassemble the guide assembly 400 at the effector assembly 300, the operator can align the disassembling tool 500 with the guide assembly 400 and operate the disassembling tool 500 to move along a first path to drive the contact portion 521 to contact with the guide assembly 400, so as to enable the effector portion 522 to rotate along the second path relative to the frame body 510. The effector portion 522 applies a force to the movable position-limiting member 420 of the guide assembly 400, so as to switch the movable position-limiting member 420 and the effector assembly 300 from the joined state to the separate state. Specifically, please refer to FIGS. 39-44. The first path in FIG. 39 is a straight path that overlaps with the axial direction of the effector assembly 300, that is, the direction shown by the axis L1 in FIG. 39, and the second path is a rotational path, that is, the direction shown by an arrow R3 in FIG. 42.

Please refer to FIG. 41. In an embodiment, the disassembling tool 500 also includes a rotation-shaft member 530. The frame body 510 and the movement mechanism 520 both have a rotation-shaft hole 540. The rotation-shaft member 530 penetrates through the rotation-shaft holes 540 at the frame body 510 and the movement mechanism 520, so as to achieve a rotatable connection between the movement mechanism 520 and the frame body 510.

The rotation connection between the frame body 510 and the movement mechanism 520 is achieved through the fitting between the rotation-shaft member 530 and the rotation-shaft hole 540. In other embodiments, the rotatable connection between the frame body 510 and the movement mechanism 520 may also be achieved through hinges, bearings or other suitable structures.

Please refer to FIGS. 39-44. In an embodiment, the effector portion 522 includes a pushing shaft 5221, which extends into the first butt joint hole 3161 of the guide assembly 400 and the second butt joint hole 3162 of the effector assembly 300, when the movement mechanism 520 moves along the second path, so as to separate the movable position-limiting member 420 of the guide assembly 400 from the first butt joint hole 3161 and the second butt joint hole 3162.

Please refer to FIGS. 39-44. When the effector portion 522 rotates along the second path relative to the frame body 510, the pushing shaft 5221 extends into the first butt joint hole 3161 and the second butt joint hole 3162 to separate the movable position-limiting member 420 of the guide assembly 400 from the first butt joint hole 3161 and the second butt joint hole 3162, so as to switch the guide assembly 400 and the effector assembly 300 from the joined state to the separate state.

Please refer to FIGS. 49 and 50. In an embodiment, the effector portion 522 includes two pushing shafts 5221. The two pushing shafts 5221 extends into two opposite recessed portions 315 at the effector assembly 300, so as to contact with the movable position-limiting member, when the movement mechanism 520 moves along the second path, and drive the movable position-limiting member to move relative to the main-body member 410, so as to switch the joining portion 411 and the effector assembly 300 from the joined state to the separate state.

When the effector portion 522 rotates along the second path relative to the frame body 510, the two pushing shafts 5221 respectively extend into the two opposite recessed portions 315 at the effector assembly 300, and drive the movable position-limiting member to move relative to the main-body member 410, so as to switch the joining portion 411 and the effector assembly 300 from the joined state to the separate state.

Please refer to FIG. 57. In an embodiment, the effector portion 522 includes a hook member 5222, which is configured to hook the movable position-limiting member 420 of the guide assembly 400, when the guide assembly 400 moves along the first path, so as to drive the movable position-limiting member 420 of the guide assembly 400 to separate from the first butt joint hole 3161 and the second butt joint hole 3162.

When the guide assembly 400 moves along the first path, the hook member 5222 can hook the movable position-limiting member 420 of the guide assembly 400 to drive the movable position-limiting member 420 of the guide assembly 400 to separate from the first butt joint hole 3161 and the second butt joint hole 3162, so as to switch the guide assembly 400 and the effector assembly 300 from the joined state to the separate state.

Specifically, please refer to FIG. 57. When the guide assembly 400 enters into the accommodation cavity 513 along the first path, it first pushes the hook member 5222 to rotate along a direction indicated by R5 in FIG. 57. When the guide assembly 400 moves in position, the hook member 5222 rotates along a direction indicated by R6 in FIG. 57 under an action of a built-in torsion spring (not shown) and/or a gravity, and is snap-fitted with the movable position-limiting member 410 of the guide assembly, moreover, a rotation angle of the hook member 5222 along the direction indicated by R6 in FIG. 57 is limited. When the guide assembly 400 exits from the accommodation cavity, the hook member 5222 drives the movable position-limiting member to move, so as to switch the guide assembly 400 to the separate state.

Please refer to FIG. 58. In an embodiment, the effector portion 522 includes a rotating rod 5223, an extendable rod 5224 and an elastic member 5225. One end of the rotating rod 5223 is in rotatable connection with the contact portion 521, and the other end of the rotating rod 5223 is in movable connection with the extendable rod 5224. The elastic member 5225 is connected between the rotating rod 5223 and the extendable rod 5224. The elastic member 5225 is configured to drive the extendable rod 5224 to extend out from the rotating rod 5224 along an axial direction of the rotating rod 5223. The extendable rod 5224 extends to join with the movable position-limiting member 420 of the guide assembly 400, when the guide assembly 400 moves along the first path, so as to drive the movable position-limiting member 420 of the guide assembly 400 to separate from the first butt joint hole 3161 and the second butt joint hole 3162.

Due to the addition of the elastic member 5225, when the guide assembly 400 moves along the first path, the elastic restoring force of the elastic member 5225 can drive the extendable rod 5224 to extend out from the rotating rod 5223 along the axial direction of the rotating rod 5223, making it easier for the extendable rod 5224 to be join with the movable position-limiting member 420 of the guide assembly 400.

Specifically, please refer to FIG. 58. When the guide assembly 400 enters into the accommodation cavity 513 along the first path, the rotating rod 5223 is first pushed to rotate along the direction shown by R7 in FIG. 58. When the guide assembly 400 continues to move along the first path and does not come into contact with the contact portion 521, the rotating rod 5223 gradually rotates and resets along a direction shown by R9 in FIG. 58 under an action of the built-in torsion spring (not shown) and/or the gravity. The extendable rod 5224 is extended under the elastic restoring force of the elastic member 5225 to join with the movable position-limiting member 420 of the guide assembly 400, and the rotation angle of the rotating rod 5223 along the direction shown by R9 in FIG. 58 is limited. When the guide assembly 400 moves to contact with the contact portion 521, the guide assembly 400 continues to move along the first path, and the guide assembly 400 pushes the contact portion 521 and the rotating rod 5223 to rotate together along a direction shown by R8 in FIG. 58, and the contact portion 521 and the rotating rod 5223 remain relatively still, so as to enable the extendable rod 5224 to drive the movable position-limiting member 420 of the guide assembly 400 to separate from the first butt joint hole 3161 and the second butt joint hole 3162.

Please refer to FIGS. 39-44. In an embodiment, when the movement mechanism 520 is triggered, the effector assembly 300 moves away from the movement mechanism 520 along the first path, and drives the movement mechanism 520 to move to a position before being triggered, so as to be triggered again. After the operator has finished using the disassembling tool 500, the movement mechanism 520 can automatically reset, so as to enable the operator to use it next time.

Please refer to FIGS. 39-44. In an embodiment, the movement mechanism 520 is in rotatable connection with the frame body 510 via a rotation-shaft member 530. A torsion spring 550 is provided at the rotation-shaft member 530. The elastic restoring force of the torsion spring 550 is configured to drive the movement mechanism 520 to rotate to the position before being triggered.

In this way, after the operator has used the disassembling tool 500, the elastic restoring force of the torsion spring 550 can drive the movement mechanism 520 to automatically reset, so as to enable the operator to use it next time. Specifically, please refer to FIG. 44, the torsion spring 550 drives the movement mechanism 520 to rotate along a direction indicated by an arrow R4 in FIG. 44, so as to automatic reset the movement mechanism 520.

Please refer to FIG. 40. In an embodiment, a guide structure 511 is provided inside the frame body 510. The guide structure 511 contacts with the effector assembly 300 during disassembling, so as to guide the effector assembly 300 to move along the first path.

Due to the addition of the guide structure 511, it is easier for the operator to accurately operate the frame body 510 and the effector assembly 300 to move relative to each other along the first path. The guide structure 511 can be a guide groove or a guide rail. Specifically, please refer to FIG. 40. The guide structure 511 in FIG. 40 is a guide groove. Left-and-right inner walls of the guide groove form left-and-right guide structures 5112, and upper-and-lower inner walls of the guide groove form upper-and-lower guide structures 5111.

Please refer to FIGS. 45 and 46. In an embodiment, a position-limiting structure 512 is provided inside the frame body 510. The position-limiting structure 512 is configured to contact with the guide assembly 400 in the separate state, so as to retain the guide assembly 400 inside the frame body 510 in the separate state, when the effector assembly 300 moves away from the frame body 510 along the first path.

Due to the addition of the position-limiting structure 512, when the operator switches the guide assembly 400 and the effector assembly 300 to a separate state by using the disassembling tool 500, and then operates the frame body 510 and the effector assembly 300 to move away from each other along the first path, the position-limiting structure 512 contacts with the guide assembly 400 in the separate state to retain the guide assembly 400 in the separate state inside the frame body 510, thereby disassembling the guide assembly 400 from the effector assembly 300.

Please refer to FIGS. 39-41. In an embodiment, the frame body 510 includes an accommodation cavity 513, and the movement mechanism 520 is arranged inside the accommodation cavity 513. The frame body 510 is provided with a first opening 514, through which the guide assembly enters into and exits from the accommodation cavity 513, so as to enable the guide assembly to move inside the accommodation cavity 513 along the first path.

On the one hand, since the movement mechanism 520 is arranged inside the accommodation cavity 513, the frame body 510 protects the movement mechanism 520. On the other hand, since the frame body 510 is provided with the first opening 514, the guide assembly 400 can enter into the accommodation cavity 513 from the first opening 514, so as to enable the guide assembly 400 and the movement mechanism 520 to contact with each other, inside the accommodation cavity 513.

Please refer to FIGS. 46 and 47. In an embodiment, the frame body 510 is further provided with a second opening 515 for taking out the guide assembly 400 from the frame body 510 after the guide assembly 400 is separated from the effector assembly 300. After disassembling the guide assembly 400, the operator can take out the guide assembly 400 from the second opening 515. Specifically, referring to FIG. 47, the operator can flip the disassembling tool 500 and pour the guide assembly 400 out from the second opening 515.

On the other hand, the embodiment also provides a surgical instrument.

Please refer to FIGS. 33-48, 59 and 60, the surgical instrument includes a handle assembly 100, a barrel assembly 200, an effector assembly 300, a guide assembly 400, a disassembling tool 500 and a packaging assembly 600.

A proximal end of the barrel assembly 200 is connected with the handle assembly 100, and a distal end of the barrel assembly 200 is connected with the effector assembly 300. The guide assembly 400 includes a main-body member 410 and a movable position-limiting member 420. The main-body member 410 includes a joining portion 411 and a guide portion 412. The joining portion 411 is joined with the end 310 of the effector assembly of the surgical instrument. The guide portion 412 is arranged at the joining portion 411. The movable position-limiting member 420 is in movable connection with the main-body member 410, and the movable position-limiting member 420 is capable of moving relative to the main-body member 410, so as to switch the joining portion 411 and the staple anvil 313 between a separate state and a joined state. The disassembling tool 500 is configured to drive the guide assembly 400 and the effector assembly 300 of the surgical instrument to switch from the joined state to the separate state. The packaging assembly 600 includes a first packaging member 610 and a second packaging member 620. The first packaging member 610 is configured to package the handle assembly 100, the barrel assembly 200 and the effector assembly 300, and the second packaging member 620 is configured to package the guide assembly 400 and the disassembling tool 500.

When it is necessary to operate on tissue in a relatively narrow space, the operator can join the joining portion 411 of the guide assembly 400 with the end of the effector assembly 300 of the surgical instrument, so as to guide the effector assembly 300 into the tissue in the narrow space through the guide portion 412 of the guide assembly 400. When the guide assembly 400 is not needed, the operator can use a disassembling tool to move the movable position-limiting member 420 to move, so as to switch the guide assembly 400 and the surgical instrument from the joined state to the separate state, and then disassemble the guide assembly 400. On the one hand, the operator is capable of satisfying a surgical requirement with only one surgical instrument with a guide assembly 400, without having to operate another instrument, which is conducive to reducing medical costs and expenses. On the other hand, the handle assembly 100, the barrel assembly 200 and the effector assembly 300 are packaged by the first packaging member 610, and the operator can use the surgical instrument normally after unpacking the first packaging member 610. When the guide assembly 400 needs to be used, the operator unpacks the second packaging 620 to use the guide assembly 400 and the disassembling tool 500.

Please refer to FIGS. 33-48, 59 and 60. In an embodiment, the second packaging member 620 is connected with an upper surface, a lower surface, a side portion or an inner portion of the first packaging member 610, so as to facilitate the operator to take out the first packaging member 610 and the second packaging member 620 at the same time.

Please refer to 33-48, 59 and 60. In an embodiment, the second packaging member 620 is connected with the first packaging member 610 via a snap-in structure of interference fit 630.

On the one hand, it is convenient for the operator to take out the first packaging member 610 and the second packaging member 620 at the same time. On the other hand, when the operator only needs to use inner components of the first packaging member 610, the first packaging member 610 can be easily separated from the second packaging member 620. Specifically, the guide assembly 400 and the disassembling tool 500 may be arranged side by side or in a stacked manner inside the second packaging member 620.

The above specific embodiments are to illustrate this disclosure, which are only used to help understanding this disclosure and are not intended to limit this disclosure. For those skilled in the art of the technical field to which this disclosure belongs, several simple deductions, deformations or substitutions can be made according to the idea of this disclosure.

## Claims

1. A disassembling tool for a guide assembly for a surgical instrument, **characterized in that**, comprising:
a frame body, which implements a forward movement and a reverse movement along a first path relative to the guide assembly for the surgical instrument and an effector assembly of the surgical instrument, while the disassembling tool disassembles the guide assembly;
a movement mechanism, which is in movable connection with the frame body; wherein the forward movement of the frame body along the first path is capable of triggering the movement mechanism to move relative to the guide assembly along a second path, and the reverse movement of the frame body along the first path is capable of triggering the movement mechanism to drive the guide assembly and the effector assembly of the surgical instrument to switch from a joined state to a separate state.

2. The disassembling tool according to claim 1, **characterized in that**, the first path is a straight path, which is parallel to a joining direction of the guide assembly, and the second path is a circular path, wherein the straight path intersects with the circular path.

3. The disassembling tool according to claim 1, **characterized in that**, when the frame body implements the forward movement along the first path, the guide assembly passes over the movement mechanism and drives the movement mechanism to implement a forward movement along the second path; when the guide assembly is in position, the guide assembly is located on one side of the movement mechanism, and the movement mechanism abuts against the effector assembly.

4. The disassembling tool according to claim 1, **characterized in that**, when the frame body implements the reverse movement along the first path, the movement mechanism abuts against a movable position-limiting member of the guide assembly and drives the movable position-limiting member to move, so as to switch the movable position-limiting member and the effector assembly from the joined state to the separate state.

5. The disassembling tool according to claim 1, **characterized in that**, the movement mechanism comprises a separating member and a powering member, the forward movement of the frame body along the first path is capable of driving the separating member to implement a forward movement along the second path, and the powering member is configured to apply a force on the separating member, so as to drive the separating member to implement a reverse movement along the second path to abut against a knife slot of the effector assembly; the reverse movement of the frame body along the first path is capable of triggering the separating member to abut against a movable position-limiting member of the guide assembly and drive the movable position-limiting member to move, so as to switch the movable position-limiting member and the effector assembly from the joined state to the separate state.

6. The disassembling tool according to claim 5, **characterized in that**, the separating member comprises a switching lever, which is provided with a connection end and an effector end, which ends are opposite with each other; wherein the connection end is in rotatable connection with the frame body, and the effector end is configured to switch the movable position-limiting member and the effector assembly from the joined state to the separate state; and/or
the powering member comprises a torsion spring, and the powering member applies a torsion force to the separating member, so as to drive the separating member to implement the reverse movement along the second path.

7. The disassembling tool according to claim 6, **characterized in that**, the effector end is provided with a hook-shaped structure, which is configured to drive the movable position-limiting member to move.

8. The disassembling tool according to claim 5, **characterized in that**, the frame body is provided with a third position-limiting portion, which is configured to limit an extreme position for the reverse movement of the separating member along the second path, so as to provide a gap between the separating member and a bottom surface of the knife slot of the effector assembly, when the separating member is inserted into the knife slot.

9. The disassembling tool according to claim 5, **characterized in that**, the separating member is provided with a fourth position-limiting portion, which abuts against the effector assembly, so as to provide a gap between the separating member and a bottom surface of the knife slot of the effector assembly, when the separating member is inserted into the knife slot.

10. The disassembling tool according to claim 1, **characterized in that**, the frame body is provided with a fifth position-limiting portion, which is configured to limit an extreme position for a reverse movement of the guide assembly along the first path, so as to position the guide assembly on an inner side of the movement mechanism.

11. The disassembling tool according to claim 1, **characterized in that**, the frame body comprises an accommodation cavity, wherein the movement mechanism is arranged inside the accommodation cavity; the frame body is provided with a first opening, through which the guide assembly enters into and exits from the accommodation cavity, so as to enable the guide assembly to move inside the accommodation cavity along the first path; wherein the frame body is further provided with a second opening, through which the guide assembly is taken out from the frame body after the guide assembly is separated from the effector assembly.

12. A surgical instrument, **characterized in that**, comprising:
a handle assembly;
a barrel assembly, wherein a proximal end of the barrel assembly is connected with the handle assembly;
an effector assembly, wherein the effector assembly is connected with an end of the barrel assembly;
a guide assembly, which is connected with the effector assembly;
a disassembling tool, which is configured to drive the guide assembly and the effector assembly of the surgical instrument to switch from a joined state to a separate state; and
a packaging assembly, which comprises a second packaging member, wherein the second packaging member is configured to package the guide assembly and the disassembling tool; wherein the second packaging member comprises an outer housing and an inner liner, wherein the outer housing is provided with an inner cavity, the inner liner is arranged inside the inner cavity of the outer housing and capable of being taken out from the inner cavity; wherein the inner liner is configured to package at least one of the guide assembly and the disassembling tool; wherein the inner liner has a sterile environment.

13. A disassembling tool for a guide assembly for a surgical instrument, **characterized in that**, comprising:
a frame body, which moves along a first path relative to the guide assembly and an effector assembly of the surgical instrument, while the disassembling tool disassembles the guide assembly;
a movement mechanism, which is in movable connection with the frame body; wherein the movement of the frame body along the first path is capable of triggering the movement mechanism to move relative to the guide assembly along a second path, so as to drive the guide assembly and the effector assembly of the surgical instrument to switch from a joined state to a separate state.

14. The disassembling tool according to claim 13, **characterized in that**, the first path is different from the second path; or
when the frame body moves relative to the effector assembly along the first path, the frame body drives the movement mechanism to contact with the effector assembly and/or the guide assembly, so as to trigger the movement mechanism; when the movement mechanism is triggered, the movement mechanism moves along the second path and applies a continuous force to a movable position-limiting member of the guide assembly, so as to switch the movable position-limiting member and the effector assembly from the joined state to the separate state.

15. The disassembling tool according to claim 13, **characterized in that**, the movement mechanism is in rotatable connection with the frame body, and the movement mechanism comprises a contact portion and an effector portion; wherein, when the frame body moves relative to the effector assembly along the first path, the frame body drives the contact portion to contact with the guide assembly, so as to enable the effector portion to rotate relative to the guide assembly along the second path; wherein the effector portion is configured to apply a force on a movable position-limiting member of the guide assembly, so as to switch the movable position-limiting member and the effector assembly from the joined state to the separate state.
